(19) Europäisches Patentamt
European Patent Office
Office européen des brevets

(11) EP 1 049 793 B1

(12) **FASCICULE DE BREVET EUROPEEN**

(45) Date de publication et mention de la délivrance du brevet:
**12.10.2005 Bulletin 2005/41**

(21) Numéro de dépôt: **99901639.7**

(22) Date de dépôt: **28.01.1999**

(51) Int Cl.[7]: **C12N 15/87**, C07C 323/60, C07K 5/062, A61K 48/00

(86) Numéro de dépôt international:
**PCT/FR1999/000162**

(87) Numéro de publication internationale:
**WO 1999/038821 (05.08.1999 Gazette 1999/31)**

(54) **COMPOSES TRANSFECTANTS SENSIBLES AUX CONDITIONS REDUCTRICES, COMPOSITIONS PHARMACEUTIQUES LES CONTENANT, ET LEURS APPLICATIONS**

FÜR REDUZIERENDE BEDINGUNGEN EMPFINDLICHE TRANSFEKTIONS-VERBINDUNGEN, PHARMAZEUTISCHE ZUSAMMENSEZUNGEN DIE DIESE ENTHALTEN UND DEREN VERWENDUNG.

TRANSFECTING COMPOSITIONS SENSITIVE TO REDUCING CONDITIONS, PHARMACEUTICAL COMPOSITIONS CONTAINING THEM, AND THEIR APPLICATIONS

(84) Etats contractants désignés:
**AT BE CH CY DE DK ES FI FR GB GR IE IT LI LU NL PT SE**
Etats d'extension désignés:
**SI**

(30) Priorité: **30.01.1998 FR 9801065**
**06.03.1998 US 77026 P**

(43) Date de publication de la demande:
**08.11.2000 Bulletin 2000/45**

(73) Titulaire: **Aventis Pharma S.A.**
**92160 Antony (FR)**

(72) Inventeurs:
- **BYK, Gérardo**
  **55513 Qyriat Ono (IL)**
- **DUBERTRET, Catherine**
  **F-92310 Sèvres (FR)**
- **PITARD, Bruno**
  **F-92330 Sceaux (FR)**
- **SCHERMAN, Daniel**
  **F-75012 Paris (FR)**

(74) Mandataire: **Peaucelle, Chantal et al**
**Cabinet Armengaud Ainé**
**3, avenue Bugeaud**
**75116 Paris (FR)**

(56) Documents cités:
**WO-A-93/05162**
**WO-A-96/10038**
**WO-A-96/25508**
**WO-A-97/18185**

- **TANG F ET AL: "Introduction of a disulfide bond into a cationic lipid enhances transgene expression of plasmid DNA." BIOCHEMICAL AND BIOPHYSICAL RESEARCH COMMUNICATIONS, (1998 JAN 6) 242 (1) 141-5. JOURNAL CODE: 9Y8. ISSN: 0006-291X., XP002080030 United States**
- **ZABNER ET AL: "CELLULAR AND MOLECULAR BARRIERS TO GENE TRANSFER BY A CATIONIC LIPID" THE JOURNAL OF BIOLOGICAL CHEMISTRY, vol. 270, 1995, pages 18997-19007, XP002080031 cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 049 793 B1

## Description

La présente invention se rapporte à un nouvel agent pour le transfert d'acides nucléiques dans les cellules. Cet agent de transfert est plus particulièrement caractérisé en ce qu'il comporte un ou plusieurs ponts disulfure sensibles aux conditions réductrices. Ce nouvel agent est utilisable pour transférer des acides nucléiques d'intérêt dans différents types cellulaires, aussi bien *in vitro* qu' *in vivo*, ou *ex vivo*.

Avec le développement des biotechnologies, la possibilité de transférer efficacement des acides nucléiques dans les cellules est devenue une nécessité. Il peut s'agir du transfert d'acides nucléiques dans des cellules *in vitro*, par exemple pour la production de protéines recombinantes, ou au laboratoire, pour l'étude de la régulation de l'expression de gènes, le clonage de gènes, ou toute autre manipulation impliquant l'ADN. Il peut également s'agir du transfert d'acides nucléiques dans des cellules *in vivo,* par exemple pour la création d'animaux transgéniques, la réalisation de vaccins, des études de marquage ou également des approches thérapeutiques. Il peut encore s'agir du transfert d'acides nucléiques dans des cellules *ex vivo*, dans des approches de greffes de moelle osseuse, d'immunothérapie ou d'autres méthodes impliquant le transfert de gènes dans des cellules prélevées d'un organisme, en vue de leur réadministration ultérieure.

Les différents vecteurs synthétiques développés à ce jour afin d'améliorer le transfert des acides nucléiques dans les cellules possèdent une diversité structurelle considérable qui reflète l'observation du fait que leur efficacité diffère selon l'application recherchée et les types cellulaires visés. Cette efficacité est largement dépendante de leur structure.

Parmi les vecteurs synthétiques développés jusqu'à présent, les lipides cationiques occupent une place importante. Ces vecteurs sont constitués d'une partie polaire, cationique, interagissant avec les acides nucléiques, et d'une partie lipidique, hydrophobe, permettant de protéger le complexe formé vis-à-vis du milieu externe. On peut citer à titre d'exemples les lipides monocationiques (DOTMA : Lipofectin®), les lipopolyamines, en particulier la dioctadécylamidoglycyl spermine (DOGS) ou la 5-carboxyspermylamide de la palmitoylphosphatidyléthanolamine (DPPES), dont la préparation a été décrite dans la demande de brevet EP 394 111, ou encore les lipides cationiques cités dans les demandes WO 96/17823 et WO 97/18185 (incorporées à la présente par référence).

De nombreuses études ont clairement indiqué que les lipides cationiques possèdent des propriétés permettant de promouvoir la transfection. Cependant, il apparaît aujourd'hui nécessaire de mettre au point des lipides cationiques ayant des structures nouvelles qui permettent d'apporter des propriétés bénéfiques additionnelles. Ainsi, il existe un besoin en lipides cationiques qui seraient plus particulièrement adaptés au passage des barrières membranaires. En effet, de nombreux obstacles s'opposent à une réelle efficacité de la transfection, parmi lesquels la difficulté pour l'acide nucléique à franchir les membranes biologiques et à pénetrer dans les compartiments cellulaires (*"Cellular and Molecular Barriers to Gene Transfer by a Cationic Lipid",* Zabner, J. et Al., J. Biol. Chem., 1995, N° 32, 18997-19007). C'est cette difficulté technique que la présente invention se propose de résoudre.

Ainsi, la présente invention concerne de nouveaux agents de transfert d'acides nucléiques qui comportent au moins une région hydrophile cationique capable de s'associer de manière non-covalente avec des acides nucléiques et au moins une région lipophile, ces régions étant reliées l'une à l'autre par l'intermédiaire d'un bras dit "espaceur", et comprenant en outre au moins un pont disulfure positionné de sorte que sa réduction entraîne une dégradation partielle de la région lipophile, ou bien positionné de sorte que sa réduction entraîne le dédoublement dudit agent de transfert lorsque celui-ci est symétrique.

Ces agents de transfert sont capables de complexer efficacement les acides nucléiques grâce à leur partie hydrophile cationique, cette interraction compactant fortement ledit acide nucléique, et la région lipophile rend cette interraction ionique insensible au milieu externe en recouvrant la particule formée d'une pellicule lipidique.

Mais en plus de ces propriétés recherchée pour la vectorisation, les agents de transfert selon l'invention possèdent une propriété de détergent extrêmement avantageuse, en générant au niveau du milieu cellulaire réducteur, du fait de la présence du (des) pont(s) disulfure, des molécules de type chaînes alkyle polyaminées qui sont des déstabilisants des membranes. En effet, les ponts disulfure sont capables de constituer des liaisons covalentes stables en milieu oxydant, et de se casser en milieu réducteur, selon le schéma suivant :

$$X\text{-}S\text{-}S\text{-}Y \rightarrow X\text{-}SH + HS\text{-}Y$$

Ce type de structure se retrouve par exemple dans certaines protéines possédant des acides aminés cystéine, et contribue à leur structure tridimensionnelle et donc à leur activité biologique. Des ponts disulfure ont par ailleurs déjà été introduits dans certaines protéines chimériques, et en particulier dans des immunotoxines, pour relier le domaine de ciblage au domaine actif.

Par "milieu réducteur", on entend au sens de l'invention un milieu réducteur naturel, par exemple le milieu intracellulaire, en particulier le cytoplasme et surtout les endosomes. Un milieu réducteur artificiel représentatif des

conditions naturelles est par exemple un milieu comportant 0,1 % à 20 % de dithiotreitol (DTT).
Par opposition, on entend par "milieu oxydant" tout milieu qui est en contact avec l'oxygène de l'atmosphère et qui ne contient pas d'agent réducteur, notamment le milieu extracellulaire. Un milieu oxydant représentatif est par exemple constitué d'une solution isotonique de chlorure de sodium 150 mM, ou d'une solution contenant 5 % de glucose.

La demanderesse a ainsi démontré que, de façon tout à fait inattendue, un ou des ponts disulfure pouvaient être introduit dans un vecteur synthétique de transfert d'acides nucléiques, en particulier de type lipide cationique et que cela n'affectait pas sa capacité de complexer les acides nucléiques en milieu non-réducteur. Elle montre aussi que les propriétés de transfert d'acides nucléiques de ces agents sont conservées, voire même améliorées. Par ailleurs, les complexes formés sont dégradés en milieu réducteur, et donc en particulier au niveau de la cellule, ce qui permet de générer des molécules détergentes rendant ainsi une plus grande quantité d'acide nucléique accessible à la machinerie de transcription cellulaire.

On entend, au sens de l'invention par "détergent" toute molécule amphiphile ayant la propriété de s'insérer dans des membranes biologiques et à les déstabiliser. Ceci résulte de la capacité des molécules amphiphiles détergentes à rompre les membranes en s'insérant dans les doubles couches phospholipidiques et en solubilisant les lipides et les protéines (*La Cellule*, Ed. Vigot et Décarie, 1988, pp.581-583).

Un autre avantage des agents de transfert selon l'invention réside également dans leur toxicité intrinsèque réduite. En effet, l'agent de transfert étant dégradé dans la cellule au niveau des ponts disulfure sensibles aux conditions réductrices, il n'exerce pas l'effet toxique observé par les vecteurs de transfert classiques. De plus, l'amélioration du passage à travers les membranes rend possible l'utilisation de doses de complexe acide nucléique/agent de transfert plus réduites, avec les conséquences bénéfiques qui en résultent sur la toxicité.

Enfin, la demanderesse a aussi mis en évidence que les propriétés de transfert sont significativement améliorées lorsque la lipophilie des agents de transfert est suffisante et qu'ils sont utilisés en quantité adéquate. Plus particulièrement, il a été montré que l'un des intérêts majeurs d'augmenter la lipophilie de ces agents, ou bien d'introduire une chaîne dérivée d'un stéroïde, est l'induction d'une résistance au sérum améliorée

Les agents de transfert selon l'invention peuvent avoir deux types de structure, sans que cela n'ait d'influence sur leur effet technique. Dans le premier cas, cette structure peut être représentée de la façon suivante :

région hydrophile cationique —— espaceur —— région lipophile

Dans une telle structure, le ou les ponts disulfure sont positionnés dans la région lipophile de façon à générer une molécule amphiphile détergente lorsqu'ils sont réduits.
Le second type de structure peut être représenté ainsi :

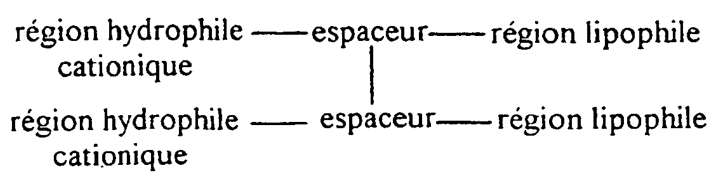


Dans ce cas, le ou les ponts disulfure sont positionnés de façon à ce que leur réduction entraîne le dédoublement des deux parties symétriques de l'agent de transfert, c'est-à-dire entre les deux parties espaceurs.

Au sens de l'invention, on entend par "partie hydrophile cationique" toute molécule hydrophile dont la charge globale est positive à pH physiologique, c'est-à-dire entre pH 5 et 8, et possédant en outre des propriétés de liaison avec les acides nucléiques. Cette liaison est en particulier du type liaison non-covalente, comme par exemple des interractions ioniques. De préférence, la région hydrophile cationique présente dans les agents de transfert selon l'invention est une polyamine ou une polyaminoguanidine.

Selon une variante avantageuse, la région hydrophile cationique répond à la formule générale suivante :

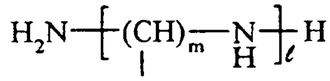

dans laquelle m est un nombre entier supérieur ou égal à 2 et ℓ est un nombre entier supérieur ou égal à 1, m pouvant

varier entre les différents groupes de carbone compris entre deux amines. Préférentiellement, m est compris entre 2 et 6 inclusivement, et ℓ est compris entre 1 et 5 inclusivement. Encore plus préférentiellement, la région polyamine est représentée par la spermine.

Une autre région polyamine préférée répond à la formule générale suivante décrite dans la demande WO 97/18185 :

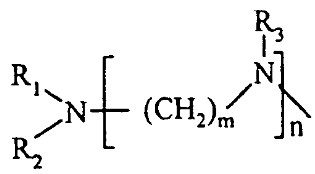

dans laquelle $R_1$, $R_2$, et $R_3$ représentent indépendamment l'un de l'autre un atome d'hydrogène ou un groupement-$(CH_2)_q$-NRR' avec q pouvant varier entre 1, 2, 3, 4, 5, et 6 ceci de manière indépendante entre les différents groupements $R_1$, $R_2$, et $R_3$, et R et R' représentent indépendamment l'un de l'autre un atome d'hydrogène ou un groupement -$(CH_2)_q$-$NH_2$ avec q défini comme précédemment, et m et n représentent indépendamment l'un de l'autre un nombre entier pouvant varier entre 0 et 6 avec, lorsque n est supérieur à 1, m pouvant prendre des valeurs différentes et $R_3$ des significations différentes au sein de la formule générale ci-dessus.

La région lipophile présente dans les agents de transfert selon l'invention est constituée d'au moins une chaîne aliphatique grasse et d'une ou plusieurs autres chaînes aliphatiques, d'un ou plusieurs dérivés de stéroïde, d'un lipide naturel ou synthétique, ou éventuellement d'une combinaison de ceux-ci, de préférence capable de former des phases lamellaires ou hexagonales. Ces structures sont caractérisées par des distances entre les lamelles ou les tubes qui dépendent de la longueur de la chaîne aliphatique grasse ou de la partie polaire du lipide.

Le terme "chaîne aliphatique grasse" désigne au sens de l'invention une chaîne alkyle ramifiée ou linéaire comprenant 10 à 22 atomes de carbone, éventuellement saturée et/ou fluorée. De préférence , elle comprend 12 à 22 atomes de carbone. On peut citer plus particulièrement les groupements aliphatiques en $C_{12}$, $C_{13}$, $C_{14}$, $C_{16}$, $C_{18}$, $C_{19}$, etc..., et notamment les groupements $(CH_2)_{11}CH_3$, $(CH_2)_{12}CH_3$, $(CH_2)_{13}CH_3$, et $(CH_2)_{17}CH_3$.

Lorsque la région lipophile comprend un dérivé d'un stéroïde, celui-ci est choisi de préférence parmi le cholestérol, l'acide cholique, ou la cholestérylamine.

Dans un mode préféré de mise en oeuvre, la région lipophile est composée de deux chaînes aliphatiques grasses au moins. Encore plus préférentiellement, elle est composée de deux ou trois chaînes aliphatiques grasses.

Selon une autre variante avantageuse de l'invention, la partie lipophile est constituée d'une chaîne aliphatique grasse et d'un dérivé de stéroïde.

Lorsque l'agent de transfert selon l'invention présente une structure symétrique, chaque partie symétrique de la molécule contient au moins une chaîne aliphatique grasse.

La région hydrophile cationique et la région lipophile peuvent être reliées entre elles par l'intermédiaire d'un bras dit "espaceur". L'espaceur peut être décrit non-limitativement comme tout groupement acide ou amine comportant des fonctions hydrolysables, connu de l'homme du métier. De manière préférée, la région dite espaceur comporte une chaîne aliphatique ou aromatique. Préférentiellement, la région espaceur peut par exemple être choisie parmi les groupements amide, carbamate, ester, éther, ou cycles aromatiques.

Les agents de transfert selon l'invention comportent un ou plusieurs ponts disulfure. Le nombre de ces ponts est déterminé par l'homme du métier en fonction de la structure de l'agent de transfert et des propriétés recherchées. Avantageusement, l'agent de transfert comporte un ou deux ponts disulfure, et de préférence un pont disulfure. Dans l'agent de transfert, le (les) pont(s) disulfure peuvent être positionnés en différents endroits. La position dépend du nombre de ponts, et de la structure de l'agent.

Selon un premier mode de réalisation, le pont disulfure est positionné de sorte que sa réduction entraîne une dégradation partielle de la région lipophile, générant ainsi au niveau de la cellule une molécule amphiphile détergente. Cette dégradation partielle peut correspondre notamment à la perte d'une chaîne aliphatique lorsque la région lipophile en compte plusieurs, ou encore à la perte de la chaîne dérivée d'un stéroïde lorsque la région lipophile en contient une. On entend par perte d'une chaîne grasse aliphatique soit la perte complète de celle-ci, soit une perte partielle, la partie restante étant alors trop courte pour constituer une chaîne grasse (longueur de moins de 10 atomes de carbone). Une telle rupture détruit l'intégrité du complexe qui se désagrège ensuite progressivement pour aboutir à des éléments dissociés dont l'un au moins est une molécule amphiphile détergente. La dégradation du complexe peut être aisément contrôlée en microscopie ou par électrophorèse.

Selon une autre variante, le pont disulfure est positionné entre les deux bras espaceurs d'un agent de transfert de structure symétrique, de sorte que sa réduction entraîne le dédoublement dudit agent de transfert.

Des agents de transfert préférés selon l'invention comprennent :

- comme région hydrophile cationique une polyamine ou polyaminoguanidine,
- comme région lipophile au moins deux chaînes aliphatiques grasses, ou bien au moins une chaîne dérivée d'un stéroïde et une chaîne aliphatique grasse, et,
- un pont disulfure dont la réduction conduit à la perte d'une chaîne aliphatique grasse, ou bien d'une chaîne dérivée d'un stéroïde lorsque l'agent de transfert en contient une.

De manière particulièrement avantageuse, les agents de transfert de l'invention comprennent une région polyamine, trois chaînes aliphatiques dont deux au moins sont grasses, et un pont disulfure conduisant, en milieu réducteur, à la perte d'une chaîne aliphatique.

D'autres agents de transfert particulièrement avantageux comprennent une région polyamine, une chaîne grasse aliphatique et une chaîne dérivée d'un stéroïde, et un pont disulfure conduisant en milieu réducteur à la perte de la chaîne dérivée d'un stéroïde.

D'autres agents de transfert particulièrement avantageux sont constitués de deux lipopolyamines symétriques, et d'un pont disulfure conduisant en milieu réducteur à leur dédoublement.

De tels agents sont illustrés dans les exemples. On peut citer à titre non-limitatif les composés suivants :

- $NH_2(CH_2)_3NH(CH_2)_4NH(CH_2)_3NHCH_2COCys[S\text{-}S\text{-}(CH_2)_4CH_3]\text{-}N[(CH_2)_{17}CH_3]_2$ :

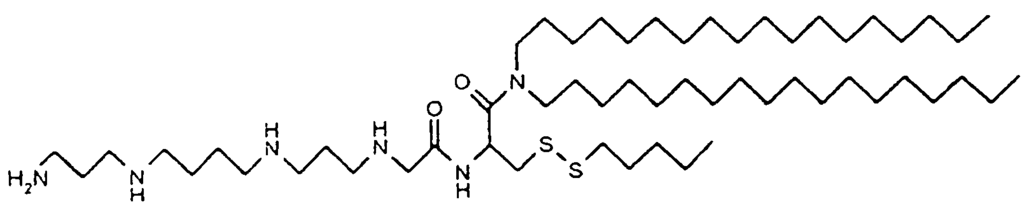

(I)

-$NH_2(CH_2)_3NH(CH_2)_4NH(CH_2)_3NHCH_2COCys[S\text{-}S\text{-}(CH_2)_{17}CH_3]\text{-}NH(CH_2)_{17}CH_3$ :

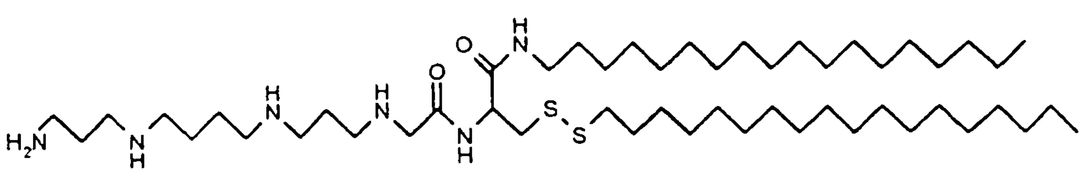

(II)

- $NH_2(CH_2)_3NH(CH_2)_4NH(CH_2)_3NHCH_2COCys[S\text{-}S\text{-}(CH_2)_{11}CH_3]\text{-}NH(CH_2)_{17}CH_3$ :

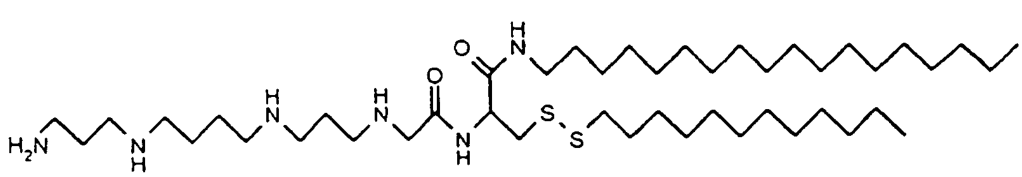

(III)

- $NH_2(CH_2)_3NH(CH_2)_4NH(CH_2)_3NHCH_2COCys[S\text{-}S\text{-}(CH_2)_{11}CH_3]\text{-}N[(CH_2)_{17}CH_3]_2$ :

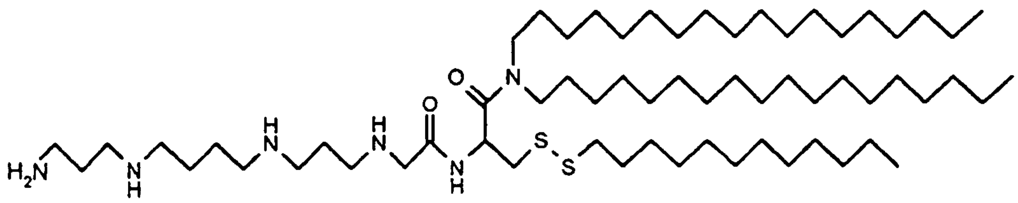

(IV)

- $NH_2(CH_2)_3NH(CH_2)_4NH(CH_2)_3NHCH_2COCys[S\text{-}S\text{-}Cholestérol]\text{-}NH(CH_2)_{17}CH_3$ :

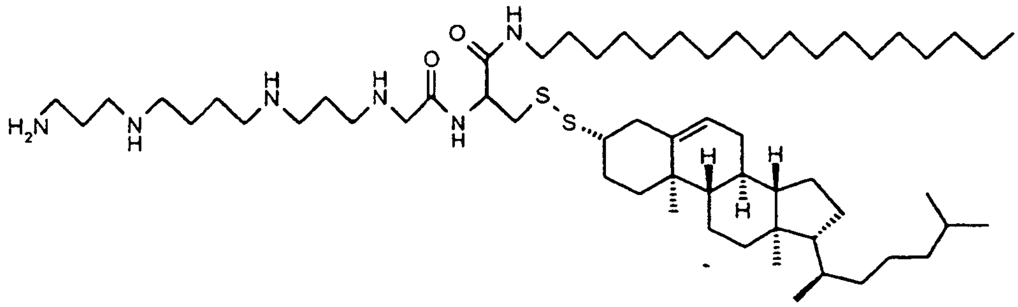

(V)

- $[NH_2(CH_2)_3NH(CH_2)_4NH(CH_2)_3NHCH_2COCysNH(CH_2)_{17}CH_3]_2$ :

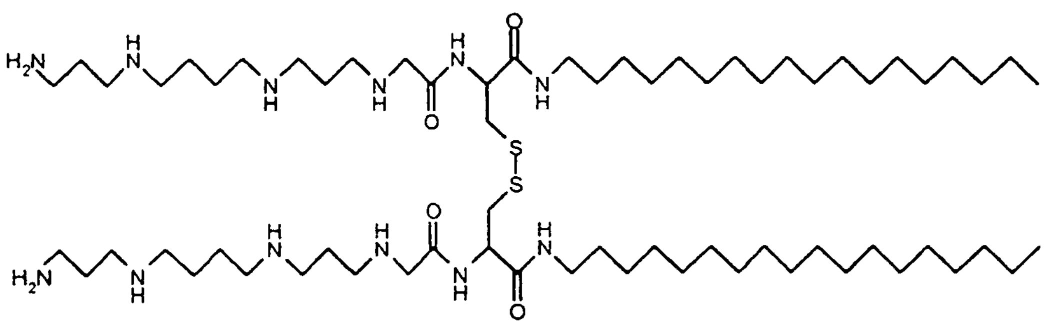

(VI)

Un autre objet de l'invention concerne une composition comprenant un agent de transfert tel que défini ci-avant, et au moins un acide nucléique. Préférentiellement, l'agent de transfert et l'acide nucléique sont présents en quantités telles que le rapport des charges positives de l'agent sur les charges négatives de l'acide nucléique soit compris entre 0,1 et 50. Ce rapport peut être ajusté aisément par l'homme du métier en fonction de l'agent utilisé, de l'acide nucléique, et du type de cellules à transfecter. Avantageusement, ce rapport se situe entre 3 et 12 nanomoles d'agent selon l'invention par µg d'acide nucléique, et de manière préférée entre 3 et 9 nanomoles d'agent transfectant par µg d'acide nucléique.

On entend au sens de l'invention par "acide nucléique" aussi bien un acide désoxyribonucléique qu'un acide ribonucléique. Il peut s'agir de séquences naturelles ou artificielles, et notamment d'ADN génomique (ADNg), d'ADN complémentaire (ADNc), d'ARN messager (ARNm), d'ARN de transfert (ARNt), d'ARN ribosomique (ARNr), de séquences hybrides ou de séquences synthétiques ou semi-synthétiques, d'oligonucléotides modifiés ou non. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement, c'est-à-dire qu'il peut s'agir d'acide pseudonucléiques (PNA), d'oligonucléotides modifiés par des diverses liaisons chimiques (par exemple phosphorothioate ou méthylphosphonate), ou encore d'oligonucléotides fonctionnalisés , c'est-à-dire couplés avec une ou plusieurs molécules ayant des propriétés caractéristiques distinctes. Concernant plus particulièrement les acides désoxyribonucléiques, ils peuvent

être simple ou double brin, de même que des oligonucléotides courts ou des séquences plus longues. En particulier, les acides nucléiques sont avantageusement constitués par des plasmides, vecteurs, épisomes, cassettes d'expression, etc. Ces acides désoxyribonucléiques peuvent porter des gènes d'intérêt thérapeutiques, des séquences régulatrices de la transcription ou de la réplication, des séquences antisens modifiées ou non, des régions de liaison à d'autres composants cellulaires, etc...

De préférence, l'acide nucléique comprend une cassette d'expression constituée d'un ou plusieurs gènes d'intérêt thérapeutique sous contrôle d'un ou plusieurs promoteurs et d'un terminateur transcriptionnel actifs dans les cellules cibles.

Au sens de l'invention, on entend par "gène d'intérêt thérapeutique" notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier une expression insuffisante dans la cellule ou l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc... Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits d'interêt thérapeutiques au sens de la présente invention, on peut citer plus particulièrement les enzymes, les dérivés sanguins, les hormones, les lymphokines [interleukines, interférons, TNF, etc... (FR 92/03120)], les facteurs de croissance, les neurotransmetteurs ou leurs précurseurs ou enzymes de synthèse, les facteurs trophiques (BDNF, CNTF, NGF, IGF, GMF, aFGF, bFGF, NT3, NT5, HARP/pléiotrophine, etc...) la dystrophine ou une minidystrophine (FR 91/11947), la protéine CFTR associée à la mucoviscidose, les gènes suppresseurs de tumeurs [p53, Rb, Rap1A, DCC, k-rev, etc... (FR 93/04745)], les gènes codant pour des facteurs impliqués dans la coagulation (Facteurs VII, VIII, IX), les gènes intervenant dans la réparation de l'ADN, les gènes suicides (thymidine kinase, cytosine déaminase), les gènes de l'hémoglobine ou d'autres transporteurs protéiques, les gènes correspondant aux protéines impliquées dans le métabolisme des lipides, de type apolipoprotéine choisie parmi les apolipoprotéines A-I, A-II, A-IV, B, C-I, C-II, C-III, D, E, F, G, H, J et apo(a), les enzymes du métabolisme comme par exemple la lipoprotéine lipase, la lipase hépatique, la lécithine cholestérol acyltransférase, la 7 alpha cholestérol hydroxylase, la phosphatidique acide phosphatase, ou encore des protéines de transfert de lipides comme la protéine de transfert des esters de cholesterol et la protéine de transfert des phospholipides, une protéine de liaisons des HDL ou encore un récepteur choisi par exemple parmi les récepteurs LDL, récepteurs des chylomicrons-remnants et les récepteurs scavenger etc...

L'acide nucléique d'intérêt thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprenent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus", d'autres virus ou encore spécifiques de tumeurs (EP 259 212).

Préférentiellement, l'acide nucléique comprend également des séquences permettant l'expression du gène d'intérêt thérapeutique et/ou du gène codant pour le peptide antigénique dans la cellule ou l'organe désiré. Il peut s'agir des séquences qui sont naturellement responsables de l'expression du gène considéré lorsque ces séquences sont susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc... En outre, ces séquences d'expression peuvent être modifiées par addition de séquences d'activation, de régulation, etc... Il peut aussi s'agir de promoteur, inductible ou répressible.

Par ailleurs, l'acide nucléique peut également comporter, en particulier en amont du gène d'intérêt thérapeutique, une séquence signal dirigeant le produit thérapeutique synthétisé dans les voies de sécrétion de la cellule cible.

Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Les compositions peuvent en outre comporter des adjuvants capables de s'associer au complexe agent de transfert/acide nucléique et d'en améliorer le pouvoir transfectant. Dans cette optique, les compositions selon l'invention peuvent comprendre comme adjuvant un ou plusieurs lipides neutres. De telles compositions sont particulièrement avantageuses, notamment lorsque le rapport de charges positives de l'agent sur les charges négatives de l'acide nucléique est faible. Il a en effet été montré que l'addition d'un lipide neutre permet d'améliorer la formation de particules nucléolipidiques et de favoriser la pénétration cellulaire en déstabilisant la membrane.

Plus préférentiellement, les lipides neutres utilisés dans le cadre de la présente invention sont des lipides à deux chaînes grasses.

De manière particulièrement avantageuse, on utilise des lipides naturels ou synthétiques, zwitterioniques ou dépourvus de charge ionique dans les conditions physiologiques. Ils peuvent être choisis plus particulièrement parmi la dioleoylphosphatidyléthanolamine (DOPE), l'oléoylpalmitoylphosphatidyléthanolamine (POPE), les di-stéaroyl, -palmitoyl, -cholestéryl, -mirystoylphosphatidyl-éthanolamines ainsi que leurs dérivés N-méthylés un à trois fois, les phosphatidylglycérols, les glycosyldiacylglycérols, les cérébrosides (tels que notamment les galactocérébrosides), les sphingolipides (tels que notamment les sphingomyélines), ou encore les asialogangliosides (tels que notamment les asialoGM1 et GM2). Ces différents lipides peuvent être obtenus soit par synthèse, soit par extraction à partir d'organes (par exemple le cerveau) ou d'oeufs, par des techniques classiques bien connues de l'homme du métier. En particulier, l'extraction des lipides naturels peut être réalisée au moyen de solvants organiques (voir également Lehninger Biochemistry).

Plus récemment, la demanderesse a démontré qu'il était également particulièrement avantageux d'employer à titre d'adjuvant, un composé intervenant ou non directement au niveau de la condensation dudit acide nucléique, tels que ceux cités dans la demande WO 96/25508. La présence d'un tel composé, au sein d'une composition transfectante à base d'une lipopolyamine, permet de diminuer considérablement la quantité de cet agent, avec les conséquences bénéfiques qui en découle sur le plan toxicologique, sans porter un préjudice quelconque à l'activité transfectante de ladite composition. Par composé intervenant au niveau de la condensation de l'acide nucléique, on entend définir un composé compactant, directement ou non, l'acide nucléique. Plus précisément, ce composé peut soit agir directement au niveau de l'acide nucléique à transfecter soit intervenir au niveau d'un composé annexe qui lui est directement impliqué dans la condensation de cet acide nucléique. De préférence, il agit directement au niveau de l'acide nucléique. Notamment, l'agent précompactant peut être tout polycation, par exemple la polylysine. Selon un mode de réalisation préféré, cet agent intervenant au niveau de la condensation de l'acide nucléique dérive, en tout ou en partie d'une protamine, d'une histone, d'une nucléoline et/ou de l'un de leurs dérivés. Un tel agent peut également être constitué, en tout ou partie, de motifs peptidiques (KTPKKAKKP) et/ou (ATPAKKAA), le nombre des motifs pouvant varier entre 2 et 10. Dans la structure du composé selon l'invention, ces motifs peuvent être répétés de manière continue ou non. C'est ainsi qu'ils peuvent être séparés par des liens de nature biochimique, par exemple un ou plusieurs acides aminés ou de nature chimique.

Préférentiellement, les compositions de l'invention comprennent de 0,01 à .20 équivalents d'adjuvant pour un équivalent d'acides nucléiques en poids/poids, et plus préférentiellement de 0,5 à 5.

Les compositions selon l'invention peuvent également mettre en oeuvre un ou plusieurs éléments de ciblage permettant de diriger les complexes nucléiques vers des recepteurs ou des ligands à la surface de la cellules. A titre d'exemple, la composition de la présente invention peut comprendre un ou plusieurs anticorps dirigés contre des molécules de la surface cellulaire, ou encore un ou plusieurs ligands de recepteurs membranaires comme l'insuline, la transferrine, l'acide folique ou tout autre facteur de croissance, cytokines ou vitamines. Avantageusement, la composition peut utiliser des lectines, modifiées ou non, afin de cibler des polysaccharides particuliers à la surface de la cellule ou sur la matrice extracellulaire avoisinante. Des protéines à motif RGD, des peptides contenant un tandem de motifs RGD, cyclique ou non, ainsi que des peptides polylysine peuvent ainsi être utilisés. Plus récemment, il a également été décrit des peptides ligands, naturels ou synthétiques, intéressants notamment pour leur sélectivité vis à vis de cellules spécifiques et capables de promouvoir efficacement l'internalisation au niveau de ces cellules ( Bary et al. Nature Medicine, 2, 1996, 299-305). Ces agents de ciblages sont généralement conjugués à l'agent de transfection cationique considéré.

L'invention s'étend également à toute composition telle que définie ci-avant et comprenant en outre un ou plusieurs autres agents connus pour transfecter l'acide nucléique. Notamment, on peut citer les produits décrits dans le brevet EP 394 111 ou dans les demandes de brevet WO 96/17823 ou WO 97/18185 (incorporées à la présente par référence).

Un autre objet de la présente invention concerne également l'utilisation d'un agent de transfert tel que défini précédemment pour le transfert d'acides nucléiques dans les cellules.

Les compositions comprenant l'agent de transfert selon l'invention peuvent être formulées en vue d'administrations par voie topique, cutanée, orale, rectale, vaginale, parentérale, intranasale, intraveineuse, intramusculaire, sous-cutanée, intraoculaire, transdermique, intratrachéale, intrapéritonéale, etc... De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutions injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée. En ce qui concerne plus particulièrement le mode d'administration, il peut s'agir soit d'une injection directe dans les tissus ou les voies circulatoires, soit d'un traitement de cellules en culture suivi de leur réimplantation par injection ou greffe.

L'invention se rapporte en outre à une méthode de transfert d'acides nucléiques dans les cellules comprenant les étapes suivantes :

(1) la mise en contact de l'acide nucléique avec un agent de transfert tel que défini ci-avant, pour former un complexe acide nucléique/agent de transfert,

(2) la mise en contact des cellules avec le complexe formé en (1).

Dans le cas d'une composition comprenant un ou plusieurs autres agents de transfection et/ou un ou plusieurs adjuvants, l'étape (1) est précédée d'une étape de mise en contact des différents agents de transfection et/ou d'une étape de mise en contact de l'agent de transfection avec le ou les adjuvants.

Les complexes agent selon l'invention/acide nucléique sont formés en mélangeant volume à volume deux solutions contenant l'une l'agent de transfection selon l'invention sous forme de micelles ou de phase lamellaire hexagonale, et l'autre l'acide nucléique à transfecter. Les complexes se forment en quelques secondes. Ils peuvent être chargés négativement, positivement, ou être neutres, en fonction de la quantité de lipide ajoutée à l'acide nucléique (Pitard B. et al., *Proc. Natl. Acad. Sci. USA,* Vol. 94, pp. 14412-14417, December 1997). Les tailles de ces complexes varient entre 50 et 300 nm en diamètre (mesuré par diffusion quasi-élastique de la lumière et par microscopie électronique à transmission). Par ailleurs, la morphologie des complexes varie avec le rapport de charge R (rapport des charges positives apportées par le lipide cationique et des charges négatives apportées par l'acide nucléique). Par exemple, les complexes chargés négativement sont entourés de molécules d'acide nucléique. Par contre, les complexes chargés positivement présentent des lipides cationiques à leur surface. Quant aux complexes neutres, ils sont colloïdalement instables. La demanderesse a ainsi montré qu'il était possible de les stabiliser par ajout d'un agent de surface non ionique en quantité suffisante. Des agents de surface préférés sont notamment les poloxamères, les polyoxyéthylène alcools, le polyoxyéthylènenonylphényléther, ou bien les PEG (polyéthylèneglycol) à tête polyéther benzylique dendritique.

L'invention se rapporte en outre à un procédé de préparation d'une composition telle que définie ci-dessus, caractérisé en ce que l'on met en contact un acide nucléique avec un agent de transfert tel que défini ci-dessus pour former un complexe acide nucléique/agent de transfert, précédé le cas échéant de la mise en contact préalable de l'agent de transfection avec un ou plusieurs adjuvants.

La mise en contact des cellules avec le complexe peut être réalisée par incubation des cellules avec ledit complexe (pour des utilisations *in vitro* ou *ex vivo*), ou par injection du complexe dans un organisme (pour des utilisations *in vivo*). L'incubation est réalisée de préférence en présence de par exemple de 0,01 à 1000 μg d'acide nucléique pour $10^6$ cellules. Pour une administration *in vivo,* des doses d'acides nucléiques allant de 0,01 à 10 mg peuvent être utilisées.

Les agents de transfert selon l'invention sont particulièrement intéressants pour leur utilisation dans le transfert d'acides nucléiques dans des cellules primaires ou dans des lignées établies. Il peut s'agir de cellules fibroblastiques, musculaires, nerveuses (neurones, astrocytes, cellules gliales), hépatiques, hématopoiétiques (lymphocytes, CD34, dendritiques, etc...), épithéliales, etc..., sous forme différenciées ou pluripotentes (précurseurs).

La présente invention fournit ainsi une méthode particulièrement avantageuse pour le traitement de maladies comprenant l'administration *in vivo, ex vivo,* ou *in vitro* d'un acide nucléique apte à corriger ladite maladie, associé à un composé selon l'invention. Plus particulièrement, cette méthode est applicable aux maladies résultant d'une déficience ou d'un manque en produit protéique ou nucléique. L'acide nucléique administré code pour ledit produit protéique ou contient ledit produit nucléique.

Outre les dispositions qui précèdent, la présente invention comprend également d'autres caractéristiques et avantages qui ressortiront des exemples et figures qui suivent, et qui doivent être considérés comme illustrant l'invention sans en limiter la portée. Notamment, la demanderesse propose à titre non-limitatif divers protocoles opératoires ainsi que des intermédiaires réactionnels susceptibles d'être mis en oeuvre pour préparer les agents de transfert selon

l'invention. Bien entendu, il est à la portée de l'homme du métier de s'inspirer de ces protocoles ou produits intermédiaires pour mettre au point des procédés analogues en vue de conduire à ces mêmes composés.

## Figures

Figure 1 : courbe représentant le profil de solubilisation de liposomes EPC/EPA (10 : 1) par le Triton X-100 par mesure de la turbidité de la solution à l'aide d'un spectrophotomètre. En absisse est portée la quantité de Triton X-100 en mM ajoutée. En ordonnée, on mesure l'absorbance de la solution contenant les liposomes.

Figure 2 : courbe représentant le profil de solubilisation de liposomes EPC/EPA (10 : 1) par le composé (VII) par mesure de la turbidité de la solution à l'aide d'un spectrophotomètre. En absisse est portée la quantité de composé (VII) en mM ajoutée. En ordonnée, on mesure l'absorbance de la solution contenant les liposomes.

Figure 3 : Activité de transfection du composé (VI) dans les cellules HepG2, en l'absence de sérum, comparativement au lipide cationique de formule $H_2N(CH_2)_3NH(CH_2)_4NH(CH_2)_3NHCH_2COGly[(CH_2)_{17}CH_3]_2$ utilisé comme agent de transfert de référence (appelé REF dans la suite de la demande).

Figure 4 : Activité de transfection du composé (I) et du composé (IV) dans les cellules HepG2 et HeLa, en présence et en l'absence de sérum, comparativement au lipide cationique de référence REF.

Figure 5 : Histogramme représentant l'activité de transfert *in vitro* dans les cellules HeLa du composé (I) sans co-lipide ou bien en présence d'un co-lipide DOPE ou cholestérol. L'axe des ordonnées représente l'expression de la luciférase en pg par puit. L'axe des absisses indique le rapport composé (I)/ADN en nmoles/µg d'ADN.

Figure 6 : Histogramme représentant l'activité de transfert *in vitro* dans les cellules HeLa du composé (IV) sans co-lipide ou bien en présence d'un co-lipide DOPE ou cholestérol. L'axe des ordonnées représente l'expression de la luciférase en pg par puit. L'axe des absisses indique le rapport composé (IV)/ADN en nmoles/µg d'ADN.

Figure 7 : Activité de transfection du composé (II) dans les cellules HepG2, en présence et en l'absence de sérum, comparativement au lipide cationique de référence REF.

Figure 8 : Histogramme représentant l'activité de transfert *in vitro* dans les cellules HeLa du composé (II) sans co-lipide ou bien en présence d'un co-lipide DOPE ou cholestérol. L'axe des ordonnées représente l'expression de la luciférase en pg par puit. L'axe des absisses indique le rapport composé (II)/ADN en nmoles/µg d'ADN.

Figure 9 : Activité de transfection du composé (V) dans les cellules HepG2 et HeLa, en présence et en l'absence de sérum, comparativement lipide cationique de référence REF.

## Exemples

### EXEMPLE 1 : SYNTHÈSES CHIMIQUES DES AGENTS DE TRANSFERT SELON L'INVENTION

A. MATÉRIEL

- La triéthylamine, la N-éthyldiisopropylamine, la dioctadécylamine, la $N_\alpha$,$N_\alpha$'-diBoccystine, et le réactif BOP sont disponibles dans le commerce.
  Il en va de même pour l'amylamine, l'octadécylamine, le penthanethiol, le dodécanethiol, l'octadécanethiol, et le thiocholestérol.

- Le $BocNH(CH_2)_3$ $NBoc(CH_2)_4$ $NBoc(CH_2)_3$ $NBocCH_2CO_2H$ a été synthétisé au laboratoire suivant le mode opératoire décrit dans la demande WO 97/18185 et dans l'article publié par Byk G., Frederic M., and Scherman D., Tetrahedron Letters (1997) 38, pp. 3219-3222.

B. MÉTHODES

**a) Analyses spectroscopiques**

Les spectres de RMN (Résonnance Magnétique Nucléaire) du proton ont été enregistrés sur des spectromètres Brucker 250 et 400 MHz.

**b) Techniques de chromatographie**

Les analyses CLHP (Chromatographie Liquide haute Performance) sont réalisées sur un appareil HITACHI équipé d'un autosampler AS-2000A, d'une pompe L-6200A, d'un détecteur UV L 4000 à 220 nm, et d'un intégrateur calculateur D 2500. La colonne utilisée, commercialisée par APPLIED BIOSYSTEMS, est en acier inoxydable 3 cm de longueur et de 4.6 mm de diamètre. Les phases mobiles sont l'eau et l'acétonitrile additionnées d'acide trifluoroacétique, et la phase stationnaire est Aquapore butyl 7 micron. Le débit varie entre 1 et 4 ml/min.

Les Chromatographies sur Couche Mince (CCM) sont effectuées sur plaque aluminium 20x20 recouvertes de gel de silice.

**c) Purification CLHP préparative**

- L'appareillage utilisé est un ensemble pour la chromatographie en phase liquide en mode gradient, permettant une détection U.V.. Cette chaîne préparative est composée de :

  Pompe A : GILSON modèle 305 équipée d'une tête 50 SC.
  Pompe B : GILSON modèle 303 équipée d'une tête 50 SC.
  Pompe injection : GILSON modèle 303 équipée d'une tête 25 SC.
  Module de pression : GILSON modèle 806.
  Mélangeur : GILSON modèle 811 C équipé d'une tête de 23 ml.
  Détecteur UV GILSON modèle 119 équipé d'une cellule préparative, et réglé à 220 nm.
  Collecteur de fraction : GILSON modèle 202 équipé de portoirs n° 21.
  Intégrateur SHIMADZU modèle C-R6A.
  Colonne : Colonne C4 (10 mm) en acier inoxydable de 25 cm de longueur et de 2,2 cm de diamètre commercialisée par VYDAC modèle 214 TP 1022.

- La solution de produit à purifier est chargée sur la colonne par la pompe d'injection au débit de 15 ml/min. Les phases mobiles sont l'eau et l'acétonitrile.

C. SYNTHÈSES CHIMIQUES

**a) Agents de transfert à chaînes grasses réductibles par leur pont disulfure**

Ces molécules sont de structure générale :

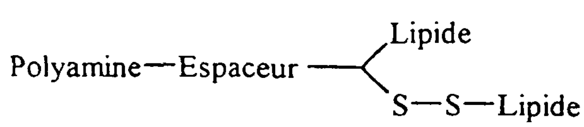

Elles ont été construites de la manière suivante :

Etape 1)

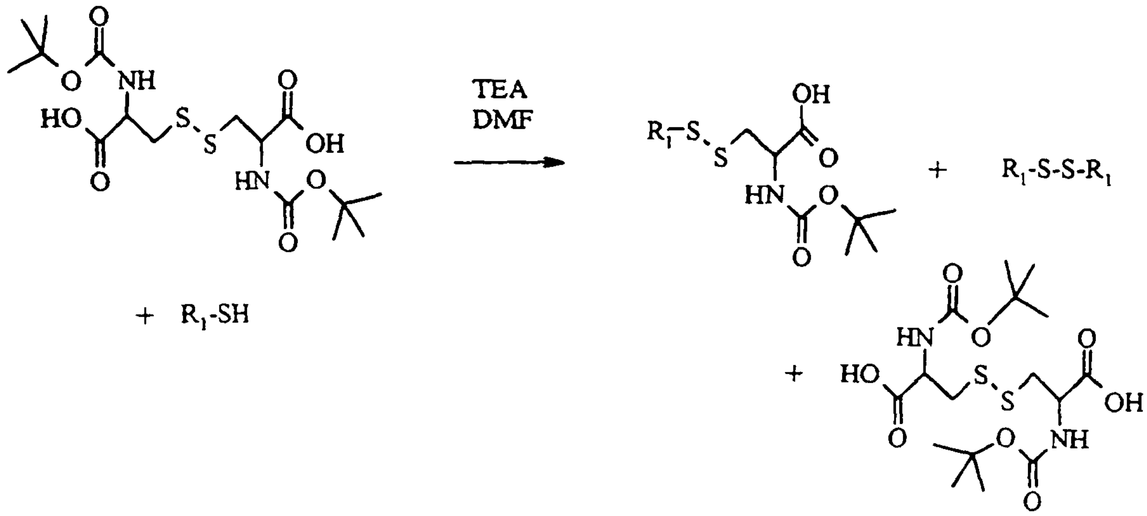


Etape 2)

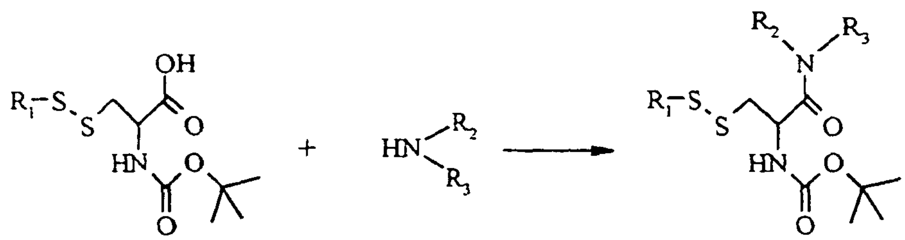

Etape 3)

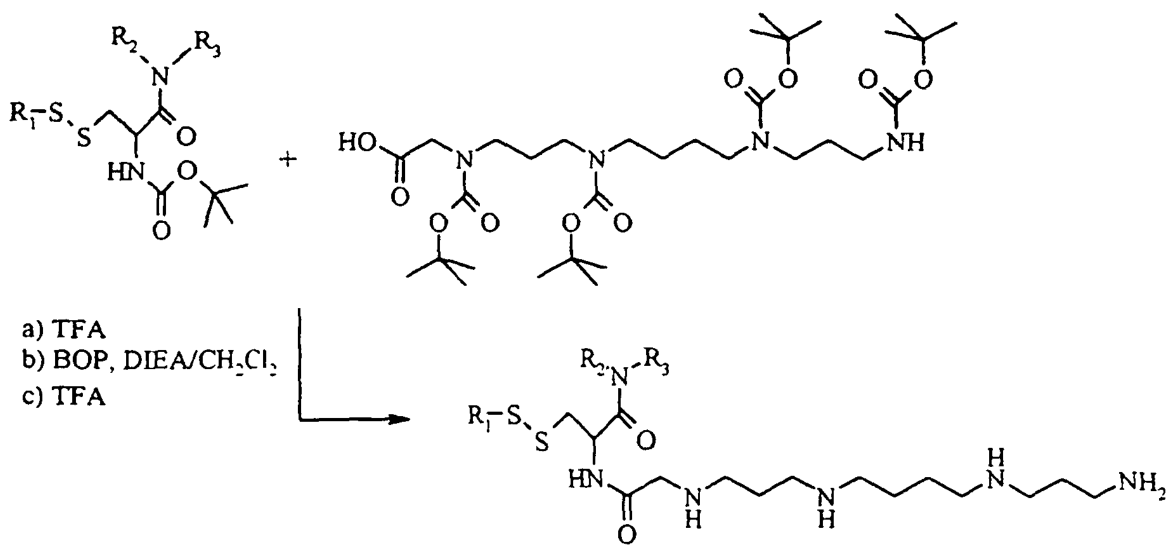

Les exemples suivants, non-limitatifs, illustrent ces agents de transfert :

composé (I) : $R_1 = (CH_2)_4CH_3$ ; $R_2 = (CH_2)_{17}CH_3$ ; $R_3 = (CH_2)_{17}CH_3$ [préparation 3.1]
composé (II) : $R_1 = (CH_2)_{17}CH_3$ ; $R_2 = (CH_2)_{17}CH_3$ ; $R_3$ = H [préparation 3.2]
composé (III) : $R_1 = (CH_2)_{11}CH_3$ ; $R_2 = (CH_2)_{17}CH_3$ ; $R_3$ = H [préparation 3.3]
composé (IV) : $R_1 = (CH_2)_{11}CH_3$ ; $R_2 = (CH_2)_{17}CH_3$ ; $R_3 = (CH_2)_{17}CH_3$ [préparation 3.4]
composé (V) : $R_1$ = Cholestéryl ; $R_2 = (CH_2)_{17}CH_3$ ; $R_3$ = H [préparation 3.5]

<u>ÉTAPE 1</u>

- PRÉPARATION 1.1 : NHBocCys[S-S-$(CH_2)_4CH_3$]-OH
  La $N_\alpha$, $N_\alpha$ '-di-Boc cystine (6,81 mmol) est dissoute dans du diméthylformamide (20 $cm^3$). A cette solution est ajoutée de la triéthylamine (58,1 mmol) puis du 1-pentanethiol (6,81 mmol). Le mélange est agité 2 heures à température ambiante. La triéthylamine est évaporée puis le concentrât est additionné à une solution de sulfate de potassium ($KHSO_4$) 0,5 M (300 $cm^3$). Le produit qui précipite est extrait par 3 fois 100 $cm^3$ de chloroforme. On réunit les phases organiques que l'on sèche sur du sulfate de magnésium anhydre, puis on évapore le chloroforme. L'extrait sec est solubilisé par de l'éther de diéthyle (100 $cm^3$) puis est extrait par 3 fois 50 $cm^3$ d'une solution de carbonate de sodium ($NaHCO_3$) saturée. Les phases aqueuses rassemblées sont neutralisées par addition jusqu'à pH = 3 d'une solution de $KHSO_4$ 0,5 M (350 $cm^3$). Le produit qui précipite est extrait par 3 fois 100 $cm^3$ de chloroforme. Les phases organiques rassemblées sont lavées par 2 fois 50 $cm^3$ d'une solution de chlorure de sodium (NaCl) saturée puis séchées sur du sulfate de magnésium anhydre. Le chloroforme est évaporé au rotoévaporateur. Le produit obtenu est élué par un mélange chloroforme/méthanol (9/1 volume/volume) sur une colonne de silice.
  On obtient 2,31 mmol de produit soit un rendement de 34%.
  CCM Rf = 0,63 ($CHCl_3$/MeOH, 9:1).

- PRÉPARATION 1.2 : NHBocCys[S-S-$(CH_2)_{17}CH_3$]-OH
  La $N_\alpha$, $N_\alpha$ '-di-Boc-cystine (6,81 mmol) est dissoute dans du diméthylformamide (20 $cm^3$). A cette solution est ajoutée de la triéthylamine (58,1 mmol) puis du 1-octadécanethiol (6,81 mmol). Le mélange est agité 2 heures à 40°C. La triéthylamine est évaporée au rotoévaporateur puis le concentrât est additionné à une solution de $KHSO_4$ 0.5 M (300 $cm^3$). Le produit qui précipite est extrait par 3 fois 100 $cm^3$ de chloroforme. On réunit les phases organiques que l'on sèche sur du sulfate de magnésium anhydre, puis on évapore le chloroforme. L'extrait sec est solubilisé par du diéthyl éther (100 $cm^3$) puis est lavé par 3 fois 50 $cm^3$ d'une solution de $NaHCO_3$ saturée. La phase éthérée est acidifiée par battages avec 2 fois 100 $cm^3$ d'une solution de $KHSO_4$ 0,5 M, puis lavée par 2 fois 50 $cm^3$ d'une solution de NaCl saturée. La phase éthérée est séchée sur du sulfate de magnésium anhydre puis est évaporée à sec au rotoévaporateur. Le produit brut obtenu est cristallisé dans de l'éther de pétrole.
  On obtient 1,36 mmol de produit soit un rendement de 20 %.
  CCM : Rf= 0,67 ($CHCl_3$/MeOH, 9:1), CLHP : Rt = 17,80 min.

- PRÉPARATION 1.3 : NHBocCys[S-S-Cholestérol]-OH
  La synthèse est identique à la préparation 1.2 mais en utilisant du thiocholestérol.
  On obtient un rendement de 58 %.
  CCM : Rf = 0,59 ($CHCl_3$/MeOH, 9:1), CLHP : Rt = 19,16 min.

- PRÉPARATION 1.4 : NHBocCys[S-S-$(CH_2)_{11}CH_3$]-OH
  La synthèse est identique à la préparation 1.2 mais à température ambiante et en utilisant du 1-dodécanethiol.
  On obtient un rendement de 40 %.
  CCM : Rf = 0,69 ($CHCl_3$/MeOH, 9:1), CLHP : Rt = 13,23 min.

<u>ÉTAPE 2</u>

- PRÉPARATION 2.1 : NHBocCys[S-S-$(CH_2)_4CH_3$]-N[$(CH_2)_{17}CH_3$]$_2$
  Le produit obtenu en 1.1 (1,15 mmol) est dissout dans du dichlorométhane (10 $cm^3$) et on ajoute N-éthyldiisopropylamine (2,86 mmol), dioctadécylamine (1,15 mmol), et BOP (1,27 mmol).

Le mélange est agité durant 2 heures et suivi par CCM et CLHP.

Le dichlorométhane est évaporé au rotoévaporateur. Le "brut" est repris dans du chloroforme (100 ml) puis lavé successivement par 3 fois 50 $cm^3$ de $KHSO_4$ 0,5 M, puis par 3 fois 50 $cm^3$ d'une solution de $NaHCO_3$ saturée, et enfin par 2 fois 50 $cm^3$ d'une solution de NaCl saturée. La phase organique est séchée sur du sulfate de magnésium anhydre, puis le chloroforme est évaporé au rotoévaporateur. On obtient un rendement de 64 %.
CCM : Rf = 0,90 ($CHCl_3$/MeOH, 9:1), CLHP : Rt = 25,96 min.

- PRÉPARATION 2.2 : NHBocCys[S-S-$(CH_2)_{17}CH_3$]-NH$(CH_2)_{17}CH_3$

  La synthèse est identique à la préparation 2.1 mais en utilisant le produit obtenu dans la préparation 1.2 comme réactif de départ.
  On obtient un rendement de 97 %.
  CCM : Rf= 0,89 ($CHCl_3$/MeOH, 9:1), CLHP : Rt = 24,84 min.

- PRÉPARATION 2.3 : NHBocCys[S-S-$(CH_2)_{11}CH_3$]-NH$(CH_2)_{17}CH_3$

  La synthèse est identique à la préparation 2.1 mais en utilisant le produit de la préparation 1.4 comme réactif de départ.
  On obtient un rendement de 86 %.
  CCM : Rf= 0,90 ($CHCl_3$/MeOH, 9:1), CLHP : Rt = 22,22 min.

- PRÉPARATION 2.4 : NHBocCys[S-S-$(CH_2)_{11}CH_3$]-N[$(CH_2)_{17}CH_3$]$_2$

  La synthèse est identique à la préparation 2.1 mais en utilisant de la dioctadécylamine et le produit de la préparation 1.4 comme réactifs de départ.
  On obtient un rendement de 85 %.
  CCM : Rf= 0,90 ($CHCl_3$/MeOH, 9:1).

- PRÉPARATION 2.5 : NHBocCys[S-S-Cholestérol]-NH$(CH_2)_{17}CH_3$

  La synthèse est identique à la préparation 2.1 mais en utilisant de l'octadécylamine et le produit de la préparation 1.3 comme réactifs de départ.
  On obtient un rendement de 90 %.
  CCM : Rf = 0,88 ($CHCl_3$/MeOH, 9:1), CLHP : Rt = 26,98 min.

ÉTAPE 3

- PRÉPARATION 3.1 [composé (I)] : $NH_2(CH_2)_3NH(CH_2)_4NH(CH_2)_3NHCH_2CO$Cys-[S-S-$(CH_2)_4CH_3$]-N[$(CH_2)_{17}CH_3$]$_2$

  L'acide trifluoroacétique (10 $cm^3$) est ajouté au produit de la préparation 2.1. Le milieu est agité durant 1,5 heures et le clivage du BOC est suivi par CLHP. L'acide trifluoroacétique est évaporé, et pour chasser les traces on évapore 3 fois 5 $cm^3$ d'éther de diéthyle.

  L'extrait sec est dissout dans 10 $cm^3$ de dichlorométhane, puis on ajoute N-éthyldiisopropylamine (3,34 mmol), BocNH$(CH_2)_3$ NBoc$(CH_2)_4$ NBoc$(CH_2)_3$ NBoc$CH_2CO_2H$ (0,644 mmol) et BOP (0,708 mmol). Le mélange est agité durant 2 heures et la réaction est suivie par CCM et CLHP.

  Le dichlorométhane est évaporé au rotoévaporateur. Le brut est repris dans du chloroforme (100 $cm^3$) puis lavé successivement par 3 fois 50 $cm^3$ de $KHSO_4$ 0,5 M, puis par 3 fois 50 ml d'une solution de $NaHCO_3$ saturée, et enfin par 2 fois 50 $cm^3$ d'une solution de NaCl saturée. La phase organique est séchée sur du sulfate de magnésium anhydre puis le chloroforme est évaporé au rotoévaporateur.

  L'acide trifluoroacétique (10 $cm^3$) est ajouté à l'extrait sec. Le milieu est agité durant 1,5 heures et le clivage des BOC est suivi par CLHP. L'acide trifluoroacétique est évaporé, et pour chasser les traces on évapore 3 fois 5 $cm^3$ d'éther de diéthyle.

  Le produit brut obtenu est purifié par CLHP préparative. Les fractions intéressantes sont regroupées et lyophilisées.

  On obtient 0,081 mmol de produit salifié soit un rendement de 11 %.

  CLHP : Rt = 17,79 min.

  Spectre de RMN $^1H$ (400 MHz, $(CD_3)_2SO$ d6, $\delta$ en ppm) : 0,90 (mt, 9H : $CH_3$ des deux octadécylamino et $CH_3$ du pentyldisulfanyl) ; de 1,15 à 1,50 (mt, 64H : 15 $CH_2$ d'un des deux octadécylamino - 15 $CH_2$ de l'autre octadécylamino) et 2 $CH_2$ du pentyldisulfanyl) ; 1,47 (mt, 2H : 1 $CH_2$ d'un des deux octadécylamino) ; de 1,55 à 1,75 (mt, 4H : 1 $CH_2$ d'un des deux octadécylamino et 1 $CH_2$ du pentyldisulfanyl) ; 1,65 (mf, 4H : les 2 $CH_2$ centraux du

butyle) ; de 1,85 à 2,05 (mt, 4H : le $CH_2$ central des deux propyles) ; de 2,70 à 2,85 (mt, 1H : 1H du $CH_2S$ de la cystéine) ; 2,78 (mt, 2H : $SCH_2$ du pentyldisulfanyl) ; de 2,85 à 3,50 (mt : les 2 $NCH_2$ du butyle - les 2 $NCH_2$ des deux propyles - l'autre H du $CH_2S$ de la cystéine et le $NCH_2$ des deux octadécylamino) ; 3,80 (s large, 2H : $NCH_2CON$ du glycylamino) ; 5,07 (mt, 1H : CONCHCON de la cystéine) ; 9,05 (d, J = 8 Hz, 1H : CONH de la cystéine) ; 7,95 - 8,85 et de 8,90 à 9,15 (respectivement 2 mis et mf étalé : les H correspondant aux NH et $NH_2$).

$MH^+$ = 969

- PRÉPARATION 3.2 [composé (II)] : $NH_2(CH_2)_3NH(CH_2)_4NH(CH_2)_3NHCH_2COCys\text{-}[S\text{-}S\text{-}(CH_2)_{17}CH_3]\text{-}NH(CH_2)_{17}CH_3$

La procédure est identique à celle décrite dans la préparation 3.1, mais en partant du produit obtenu dans la préparation 2.2.

On obtient un rendement de 31 % en produit salifié.

CLHP : Rt = 15,63 min.

Spectre de RMN $^1H$ (400 MHz, $(CD_3)_2SO$ d6, $\delta$ en ppm) : 0,89 (t, J = 7,5 Hz, 6H : $CH_3$ de l'octadécylamino et $CH_3$ de l'octadécyldisulfanyl) ; de 1,15 à 1,45 (mt, 60H : 15 $CH_2$ de l'octadécylamino et 15 $CH_2$ de l'octadécyldisulfanyl) ; 1,42 (mt : 1 des $CH_2$ de l'octadécylamino) ; de 1,55 à 1,70 (mt, 2H : 1 des $CH_2$ de l'octadécyldisulfanyl) ; 1,66 (mf, 4H : les 2 $CH_2$ centraux du butyle) ; de 1,85 à 2,05 (mt, 4H : le $CH_2$ central des deux propyles) ; 2,76 (t, J = 7,5 Hz, 2H : $SCH_2$ de l'octadécyldisulfanyl) ; de 2,85 à 3,10 (mt, 14H : les 2 $NCH_2$ du butyle - les 2 $NCH_2$ des deux propyles - 1H du $NCH_2$ de l'octadécylamino et 1H du $CH_2S$ de la cystéine) ; 3,10 (dd, J = 13,5 et 6 Hz, 1H : l'autre H du $CH_2S$ de la cystéine) ; 3,18 (mt, 1H : l'autre H du $NCH_2$ de l'octadécylamino) ; 3,82 (AB très limite, 2H : $NCH_2CON$ du glycylamino) ; 4,60 (mt, 1H : CONCHCON de la cystéine) ; 8,27 (t, J = 5,5 Hz, 1H : CONH de l'octadécylamino) ; 8,90 (d, J = 8,5 Hz, 1H : CONH de la cystéine) ; 7,95 - 8,82 et 9,07 (3 mfs : les H correspondant aux NH et $NH_2$).

$MH^+$ = 899

- PRÉPARATION 3.3 [composé (III)] : $NH_2(CH_2)_3NH(CH_2)_4NH(CH_2)_3NHCH_2COCys\text{-}[S\text{-}S\text{-}(CH_2)_{11}CH_3]\text{-}NH(CH_2)_{17}CH_3$

La procédure est identique à celle décrite dans la préparation 3.1, mais en partant du produit obtenu dans la préparation 2.3.

On obtient un rendement de 26,5 % en produit salifié.

CLHP : Rt = 12,36 min.

Spectre de RMN $^1H$ (400 MHz, $(CD_3)_2SO$ d6, $\delta$ en ppm) : 0,90 (t, J = 7,5 Hz, 6H : $CH_3$ de l'octadécylamino et $CH_3$ du dodécyldisulfanyl) ; de 1,15 à 1,50 (mt, 48H : 15 $CH_2$ de l'octadécylamino et 9 $CH_2$ du dodécyldisulfanyl) ; 1,43 (mt : 1 $CH_2$ de l'octadécylamino) ; de 1,55 à 1,70 (mt, 2H : 1 $CH_2$ du dodécyldisulfanyl) ; 1,65 (mf, 4H : les 2 $CH_2$ centraux du butyle) ; de 1,85 à 2,05 (mt, 4H : le $CH_2$ central des deux propyles) ; 2,76 (t, J = 7,5 Hz, 2H : $SCH_2$ du dodécyldisulfanyl) ; 2,80 à 3,05 (mt, 14H : les 2 $NCH_2$ du butyle - les 2 $NCH_2$ des deux propyles - 1H du $NCH_2$ de l'octadécylamino et 1H du $CH_2S$ de la cystéine) ; 3,11 (dd, J = 13,5 et 6 Hz, 1H : l'autre H du $CH_2S$ de la cystéine) ; 3,17 (mt, 1H : l'autre H du $NCH_2$ de l'octadécylamino) ; 3,83 (AB limite, 2H : $NCH_2CON$ du glycylamino) ; 4,60 (mt, 1H : CONCHCON de la cystéine) ; 8,25 (t, J = 5,5 Hz , 1H : CONH de l'octadécylamino) ; 8,99 (d, J = 8,5 Hz, 1H : CONH de la cystéine) ; 7,96 - 8,84 et 9,09 (3 mfs : les H correspondant aux NH et $NH_2$).

$MH^+$ = 815

- PRÉPARATION 3.4 [composé (IV)] : $NH_2(CH_2)_3NH(CH_2)_4NH(CH_2)_3NHCH_2COCys\text{-}[S\text{-}S\text{-}(CH_2)_{11}CH_3]\text{-}N[(CH_2)_{17}CH_3]_2$

Le produit obtenu par la préparation 2.4 est engagé dans une synthèse identique à la préparation 3.1.

On obtient un rendement de 39 % en produit salifié.

CLHP : Rt = 19,75 min.

Spectre de RMN $^1H$ (400 MHz, $(CD_3)_2SO$ d6, $\delta$ en ppm) : 0,87 (t, J = 7,5 Hz, 9H : $CH_3$ des deux octadécylamino et $CH_3$ du dodécyldisulfanyl) ; de 1,15 à 1,50 (mt, 78H : 15 $CH_2$ d'un des deux octadécylamino - 15 $CH_2$ de l'autre octadécylamino) et 9 $CH_2$ du dodécyldisulfanyl) ; 1,47 (mt, 2H : 1 $CH_2$ d'un des deux octadécylamino) ; de 1,50 à 1,70 (mt, 4H : 1 $CH_2$ d'un des deux octadécylamino et 1 $CH_2$ du dodécyldisulfanyl) ; 1,68 (mf, 4H : les 2 $CH_2$ centraux du butyle) ; de 1,85 à 2,10 (mt, 4H : le $CH_2$ central des deux propyles) ; 2,77 (t, J = 7,5 Hz, 2H : $SCH_2$ du dodécyldisulfanyl) ; 2,80 (mt, 1H : 1H du $CH_2S$ de la cystéine) ; de 2,70 à 3,50 (mt : les 2 $NCH_2$ du butyle - les 2 $NCH_2$ des deux propyles - l'autre H du $CH_2S$ de la cystéine et le $NCH_2$ des deux octadécylamino) ; 3,80 (s large, 2H : $NCH_2CON$ du glycylamino) ; 5,05 (mt, 1H : CONCHCON de la cystéine) ; 9,07 (d, J = 8 Hz, 1H : CONH de la cystéine) ; de 7,75 à 8,20 et de 8,65 à 9,25 (2 mfs étalés : les H correspondant aux NH et $NH_2$).

$MH^+$ = 1067

- PRÉPARATION 3.5 [composé (V)] : $NH_2(CH_2)_3NH(CH_2)_4NH(CH_2)_3NHCH_2COCys$-[S-S-Cholesterol]-NH $(CH_2)_{17}CH_3$

  Le produit obtenu par la préparation 2.5 est engagé dans une synthèse identique à la préparation 3.1 sauf pour le clivage final des BOC pour lequel on utilise le mélange suivant : 10 $cm^3$ de TFA, 0,5 $cm^3$ d'eau, 0,5 $cm^3$ de thioanisol, et 0,75 g de phénol.

  On obtient un rendement de 5,6 % en produit salifié

  CLHP :Rt = 16,59 min.

  Spectre de RMN $^1H$ (400 MHz, $(CD_3)_2SO$ d6, à une température de 383 K, $\delta$ en ppm) : 0,74 et 1,05 (2 s, 3H chacun : $CH_3$ en 18 et $CH_3$ en 19 du cholestéryle) ; de 0,80 à 0,95 (mt : les H correspondant au $CH_3$ de l'octadécylamino et aux $CH_3$ en 26 - $CH_3$ en 27 et $CH_3$ en 21 du cholestéryle) ; 1,77 (mt : les 4H correspondant aux 2 $CH_2$ centraux du butyle) ; de 1,85 à 2,10 (mt : les 4H correspondant au $CH_2$ central des deux propyles) ; de 2,90 à 3,25 (mt : les 16H correspondant aux 2 $NCH_2$ du butyle - aux 2 $NCH_2$ des deux propyles - au $CH_2S$ de la cystéine et au $NCH_2$ de l'octadécylamino) ; 3,63 (AB limite, 2H : $NCH_2CON$ du glycylamino) ; 4,61 (mt, 1H : CONCHCON de la cystéine) ; 5,39 (mt, 1H : CH en 6 du cholestéryle) ; 7,69 (mt, 1H : CONH de l'octadécylamino) ; 8,25 (mf, 1H : CONH de la cystéine). Pour tous les autres protons du cholestéryle et de l'octadécylamino, les signaux correspondant sortent entre 0,60 et 3,00 ppm.

  $MH^+$ = 1015

**b) Agents de transfert symétriques dédoublables par pont disulfure**

Ces molécules de structure générale :

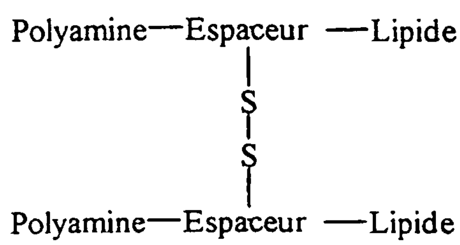

ont été construites de la manière suivante :

Etape 1)

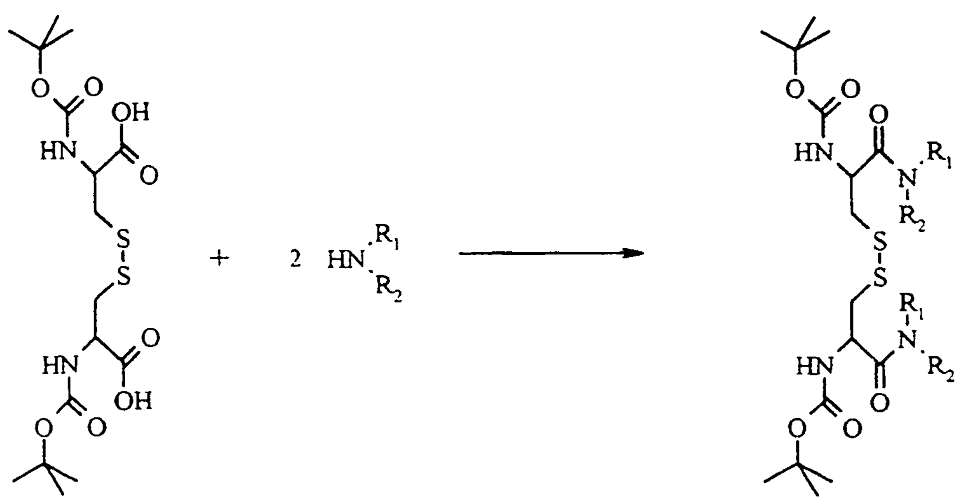

Etape 2)

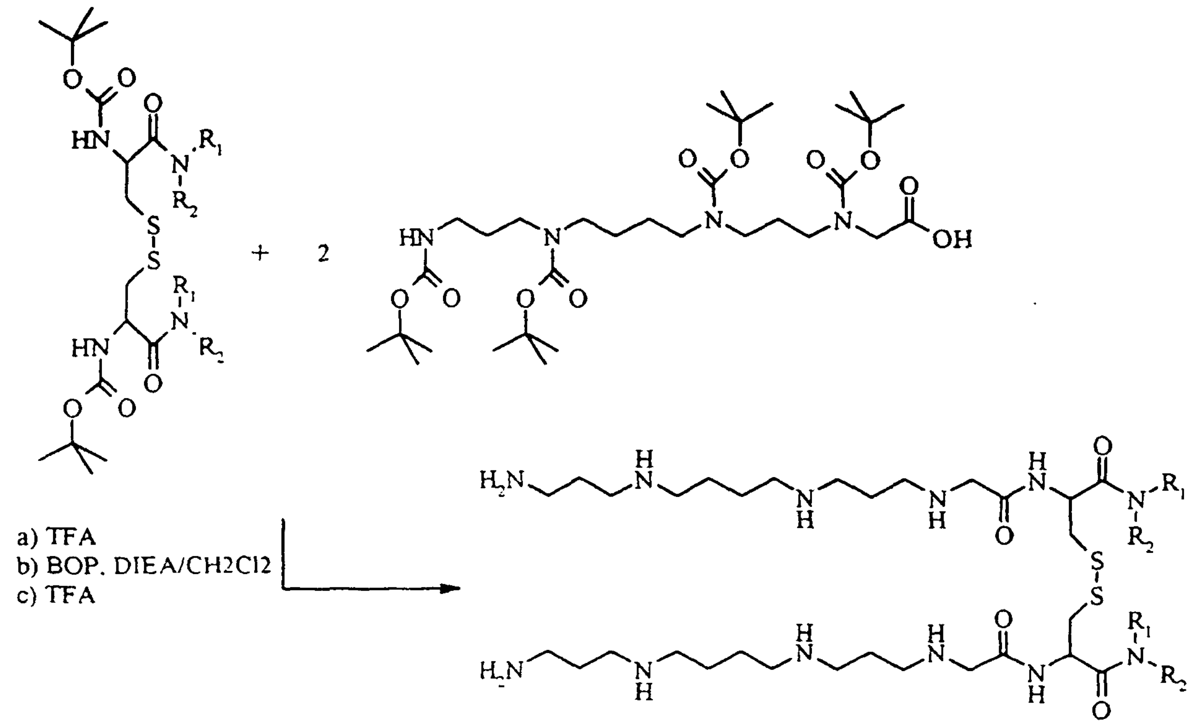


L'exemple suivant, donné à titre non-limitatif, illustre un de ces agents de transfert : composé (VI) : $R_1 = (CH_2)_{17}CH_3$ ; $R_2 = H$

ÉTAPE 1

- PREPARATION 1 : $[NHBoc-CH(CO-NH(CH_2)_{17}CH_3)CH_2-S-]_2$

  La $N_\alpha$, $N_\alpha$'-diBoc cystine (0,57 mmol) est dissoute dans du chloroforme (15 $cm^3$) et on ajoute N-éthyldiisopropylamine (5,67 mmol), octadécylamine (0,10 mmol) et BOP (1,24 mmol).

  Le mélange est agité durant 2 heures et suivi par CCM et CLHP.

  Le chloroforme est évaporé au rotoévaporateur. Le "brut" est repris dans de l'acétate d'éthyle (100 $cm^3$) puis lavé successivement par 3 fois 50 $cm^3$ de $KHSO_4$ 0,5 M, puis par 3 fois 50 $cm^3$ d'une solution de $NaHCO_3$ saturée, et enfin par 2 fois 50 $cm^3$ d'une solution de

  NaCl saturée. La phase organique est séchée sur du sulfate de magnésium anhydre puis l'acétate d'éthyle est évaporé au rotoévaporateur.

  On obtient 0,346 mmol de produit soit un rendement de 69 %.

  CCM : Rf = 0,94 ($CHCl_3$/MeOH, 9:1).

ÉTAPE 2

- PRÉPARATION 2 [composé (VI)] : $[NH_2(CH_2)_3NH(CH_2)_4NH(CH_2)_3NHCH_2CO-CyNH(CH_2)_{17}CH_3]_2$

  L'acide trifluoroacétique (5 $cm^3$) est ajouté au produit obtenu par la préparation 1. Le mélange est agité durant 1,5 heures et le clivage du BOC est suivi par CLHP. L'acide trifluoroacétique est évaporé, et pour chasser les traces on évapore 3 fois 5 $cm^3$ d'éther de diéthyle.

  L'extrait sec est dissout dans du dichlorométhane (25 $cm^3$), puis on ajoute N-éthyldiisopropylamine (3,44 mmol), $BocNH(CH_2)_3$ $NBoc(CH_2)_4$ $NBoc(CH_2)_3$ $NBocCH_2CO_2H$ (0,697 mmol) et BOP (0,86 mmol). Le mélange est agité durant 2 heures et la réaction est suivie par CCM et CLHP.

  Le dichlorométhane est évaporé au rotoévaporateur. Le "brut" est repris dans de l'acétate d'éthyle (100 $cm^3$)

puis est lavé successivement par 3 fois 50 $cm^3$ de $KHSO_4$ 0,5 M, puis par 3 fois 50 $cm^3$ d'une solution de $NaHCO_3$ saturée, et enfin par 2 fois 50 $cm^3$ d'une solution de NaCl saturée. La phase organique est séchée sur du sulfate de magnésium anhydre puis l'acétate d'éthyle est évaporé au rotoévaporateur.

CCM : Rf = 0,90 ($CHCl_3$/MeOH, 9:1), CLHP : Rt = 26,10 min.

L'acide trifluoroacétique (5 $cm^3$) est ajouté à l'extrait sec. Le mélange est agité durant 1,5 heures et le clivage des BOC est suivi par CLHP. L'acide trifluoroacétique est évaporé, et pour chasser les traces on évapore 3 fois 5 $cm^3$ d'éther de diéthyle.

Le produit brut obtenu est purifié par CLHP préparative. Les fractions intéressantes sont regroupées et lyophilisées.

On obtient 0,099 mmol de produit salifié soit un rendement de 28,5 %.

CLHP : Rt = 10,55 min.

Spectre de RMN $^1H$ (400 MHz, $(CD_3)_2SO$ d6, δ en ppm) : 0,91 (t, J = 7,5 Hz, 6H : $CH_3$ des deux octadécylamino) ; de 1,10 à 1,40 (mt, 60H : 15 $CH_2$ d'un des deux octadécylamino et 15 $CH_2$ de l'autre octadécylamino) ; 1,43 (mt, 4H : 1 $CH_2$ des deux octadécylamino) ; 1,64 (mf, 8H : les 2 $CH_2$ centraux des deux butyles) ; de 1,85 à 2,10 (mt, 8H : le $CH_2$ central des quatre propyles) ; de 2,80 à 3,15 (mt, 32H : les 2 $NCH_2$ des deux butyles - les 2 $NCH_2$ des quatre propyles - le $NCH_2$ des deux octadécylamino et le $CH_2S$ des deux cystéines) ; 3,84 (mf, 4H : le $NCH_2CON$ des deux glycylamino) ; 4,60 (mt, 2H : le CONCHCON des deux cystéines) ; 8,27 (mf, 2H : le CONH des deux octadécylamino) ; 8,95 (mf, 2H : le CONH des deux cystéines) ; 7,97 - 8,87 et 9,15 (3 mfs : les H correspondant aux NH et $NH_2$).

$MH^+$ = 1227.

## EXEMPLE 2 : ÉVALUATION DE L'ACTION DÉTERGENTE DES AGENTS DE TRANSFERT SELON L'INVENTION

L'objectif de cet exemple est de montrer que les agents de transfert selon l'invention possèdent des propriétés détergentes, c'est-à-dire qu'ils sont capables de dissoudre les membranes.

Pour cela, on utilise un modèle vitro représentant les membranes biologiques, à savoir des liposomes EPC/EPA (phosphatidylcholine d'oeuf/acide phosphatidyle d'oeuf, 10 : 1). Tout comme les membranes biologiques, les parois de ces liposomes sont constituées de bicouches de phospholipides, et elles possèdent donc un comportement comparable.

Ces liposomes sont formés par mise en solution des différents constituants dans du chloroforme, puis évaporation dudit chloroforme à l'aide d'un évaporateur rotatif. Le film lipidique obtenu est redispersé dans l'eau, puis les liposomes sont formés par sonication et chauffage.

L'évaluation de l'action détergente du produit ajouté aux liposomes est faite par mesure de la turbidité à l'aide d'un spectrophotomètre.

A titre de référence, une première expérience a été effectuée avec du Triton X-100 qui un détergent commercial bien connu. On obtient alors une solubilisation complète des liposomes (100 % de solubilisation) comme cela est représenté sur la courbe de la figure 1.

Le second produit testé est une molécule amphiphile comprenant une polyamine liée via un espaceur à une chaîne grasse contenant 18 atomes de carbone (composé (VII)). Il s'agit donc de la molécule obtenue par réduction du pont disulfure du composé (II) de la présente invention. La courbe représentée à la figure 2 montre les résultats obtenus lorsqu'on ajoute cette molécule amphiphile aux liposomes : on observe une solubilisation partielle qui correspond à une solubilisation d'environ 30 % par rapport au Triton X-100

Enfin, la même expérience a été conduite avec le lipide cationique de référence REF, à savoir l'analogue du composé (II) mais ne contenant pas de pont disulfure. Aucune solubilisation des membranes liposomiales n'a été observée.

En conclusion, cet exemple montre que les agents de transfert selon l'invention sont capables de générer en milieu réducteur des molécules amphiphiles ayant une action détergente, c'est-à-dire capable de dissoudre les membranes. Cette propriété est extrêmement avantageuse car les agents de transfert de l'invention permettent de vectoriser des acides nucléiques en plus grande quantité et plus facilement jusqu'aux compartiments cellulaires, ce qui permet une amélioration de l'efficacité de transfection (comme cela est montré dans les exemples de transfection qui suivent).

## EXEMPLE 3 : UTILISATION DES PRODUITS SELON L'INVENTION POUR LA TRANSFECTION IN VITRO DE MATÉRIEL GÉNÉTIQUE

Ces essais illustrent la capacité des agents de transfert selon l'invention à transfecter efficacement des cellules in vitro malgré l'insertion de pont(s) disulfure dans leur structure.

A. MATÉRIEL GÉNÉTIQUE UTILISÉ

Le plasmide utilisé est décrit dans le brevet WO 97/10343. Cette construction pCOR_pXL2774 comporte le gène codant pour la luciférase sous promoteur du gène très précoce du Cytomegalovirus humain [hCMV-IE].
Les solutions d'acide nucléique sont diluées à 20 µg/ml dans du sérum physiologique (NaCl 0,15 M).

B. SOLUTIONS CYTOFECTANTES (préparation extemporanée)

Les produits décrits dans l'invention sont mis en solution dans l'eau à concentration variant de 40 à 160 µmol et mélangés volume à volume avec la solution d'ADN. La concentration saline finale est de 75 mmol.

C. TRANSFECTION

Les cellules sont cultivées dans les conditions appropriées en microplaques de 24 puits (2 $cm^2$/puits) et sont transfectées alors qu'elles sont en phase exponentielle de croissance et à 50-70 % de la confluence.
Les cellules sont lavées par 2 fois 0,5 $cm^3$ de milieu dépourvu de protéines sériques et remises en croissance soit en milieu sans sérum (transfection en absence de sérum), soit en milieu complet (transfection en présence de sérum). 0,05 $cm^3$ de mélange cytofectant (0,5 µg ADN/puits) sont ajoutés aux cellules (3 puits/condition vecteur-ADN). Quand les cellules sont transfectées en l'absence de sérum, le milieu de croissance est supplémenté 2 heures après la transfection par la quantité appropriée de sérum.
L'efficacité de transfection est évaluée à 48 heures post-transfection par une mesure de l'expression de la luciférase selon les recommandations données pour l'utilisation du kit Promega (Luciférase Assay System). La toxicité des mélanges cytofectants est estimée par une mesure des concentrations protéiques dans les lysats cellulaires.

D. RÉSULTATS

**a) Agents de transfert symétriques dédoublables par réduction d'un pont disulfure : composé (VI)** (figure 3)

Ce produit décrit dans l'invention a été utilisé comparativement au lipide cationique de référence REF (décrit comme étant le composé (6) dans la demande de brevet WO 97/18185) comme vecteur d'ADN, pour transfecter la lignée cellulaire HepG2.
Pour ce type cellulaire, la toxicité du composé (VI) est du même ordre de grandeur que celle du lipide cationique de référence REF : quand la transfection est effectuée en l'absence de protéines sériques, la survie est de 80 % pour les cellules HepG2, pour des doses de 160 µM en lipide cationique.
L'efficacité maximum de transfection est obtenue pour un ratio de lipide cationique/µg d'ADN de 4 à 8 nanomoles. L'expression du transgène obtenue avec l'utilisation du composé (VI) comparativement à celle obtenue avec le lipide cationique de référence REF est supérieure (4 fois) pour des transfections de cellules HepG2

**b) Agents de transfert dont la partie lipidique est composée de deux chaînes alkyles en $C_{18}$ et d'une troisième chaîne alkyle liée par un pont disulfure : composés (I) et (IV)**

Ces deux produits décrits dans la présente invention ne présentent pas de toxicité significative jusqu'à 160 µM en lipide cationique, aussi bien pour des cellules HeLa que pour des cellules HepG2.
Par rapport aux expressions du transgène obtenues avec le lipide cationique de référence REF, l'addition d'une troisième chaîne lipidique en $C_5$ [composé (I)] permet d'obtenir des résultats de transfection en l'absence de protéines sériques du même ordre de grandeur. Par contre, dans les mêmes conditions de transfection, si la troisième chaîne lipidique est en $C_{12}$ [composé (IV)], l'expression du transgène est augmentée d'un facteur d'environ 2 fois pour les cellules HeLa et d'environ 9 fois pour les cellules HepG2 (voir figure 4).
De plus, un des intérêts majeurs de l'augmentation de la lipophilie des lipides cationiques par addition d'une troisième chaîne alkyle est mis en évidence dans des expériences de transfection en présence de protéines sériques. Dans ce cas, il n'y a en effet aucune inhibition significative due à la présence des protéines sériques, ce qui en fait des candidats de choix pour des transfections *in vivo*.
Les figures 5 et 6 représentent sous forme d'histogrammes l'efficacité de transfection des composés (I) et (IV).

**c) Agents de transfert dont la partie lipidique est composée de deux chaînes alkyles en $C_{18}$ dont l'une est liée par un pont disulfure : composé (II)**

Ce produit décrit dans l'invention ne présente pas de toxicité significative aux doses utilisées vis à vis des

cellules HepG2 (160 µM en lipide cationique).
Pour des transfections en absence de protéines sériques, le niveau d'expression du transgène est jusqu'à 3 fois suprérieur par rapport au lipide cationique de référence REF (voir figure 7).
De plus, la transfection est nettement améliorée (jusqu'à 40 fois) en présence de protéines sériques. Donc, de façon tout à fait inattendue, il n'y a aucun effet inhibiteur dû à la présence de sérum.
La figure 8 représente sous forme d'histogramme l'efficacité de transfection du composés (II).

**d) Agents de transfert dont la partie lipidique contient une chaîne dérivée d'un stéroïde liée par un pont disulfure : composé (V)** (figure 9)

Ce composé décrit dans l'invention ne présente pas de toxicité significative aux doses utilisées vis-à-vis des cellules HeLa ou HepG2 (160 µM en lipide cationique).
La liaison d'un cholestérol au lieu d'une chaîne alkyle apporte un gain très significatif quant à l'expression du transgène et de plus, dans ce cas, aucune inhibition n'a pu être constatée en présence de protéines sériques, ce qui rend ce produit très attractif pour une utilisation en transfection *in vivo*.
En conclusion, les résultats présentés dans les tableaux et histogrammes des figures 3 à 9 montrent que :

- l'introduction de pont(s) disulfure dans des agents de transfert de type lipide cationique n'affecte pas la capacité de ces agents à transfecter de l'ADN in vitro, mais conduit bien au contraire à une amélioration de l'efficacité de transfection,
- les agents de transfert selonl'invention ne sont pas toxiques aux doses utilisées,
- et enfin, l'augmentation de la lipophilie des agents de transfert selon l'invention permet de s'affranchir au moins en partie de l'inhibition de la transfection due au sérum.

## Revendications

**1.** Agent de transfert d'acides nucléiques comprenant :

- au moins une région hydrophile cationique capable de s'associer de manière non-covalente avec des acides nucléiques et,
- au moins une région lipophile constituée d'au moins une chaîne grasse aliphatique et d'une ou plusieurs chaînes aliphatiques, d'un ou plusieurs dérivés de stéroïde, d'un lipide naturel ou synthétique, et/ou éventuellement d'une combinaison de ceux-ci,

ces régions étant reliées l'une à l'autre par l'intermédiaire d'un groupement acide ou amine comportant des fonctions hydolysables, et comprenant en outre au moins un pont disulfure **caractérisé en ce que** ladite région hydrophile cationique est une polyamine ou une polyaminoguanidine et que ledit pont disulfure est positionné dans la région lipophile de façon à générer une molécule amphiphile détergente lorsqu'il est réduit, ou bien positionné de sorte que sa réduction entraîne le dédoublement dudit agent de transfert lorsque celui-ci est symétrique.

**2.** Agent de transfert selon la revendication 1 **caractérisé en ce que** la région lipophile est constituée de deux chaînes grasses aliphatiques au moins.

**3.** Agent de transfert selon la revendication 1 **caractérisé en ce que** la région lipophile est constituée d'une chaîne grasse aliphatique et d'un dérivé de stéroïde.

**4.** Agent de transfert selon la revendication 1 **caractérisé en ce que** la ou les chaînes grasses aliphatiques sont des chaînes alkyles ramifiées ou linéaires comprenant 10 à 22 atomes de carbone, éventuellement saturées et/ou fluorées.

**5.** Agent de transfert selon la revendication 1 **caractérisé en ce que** le dérivé de stéroïde est choisi parmi le cholestérol, l'acide cholique, et la cholestérylamine.

**6.** Agent de transfert selon la revendication 1 **caractérisé en ce que** le groupement acide ou amine comportant des fonctions hydolysables est choisi parmi les groupements amide, carbamate, ester, éther, et/ou les cycles aromatiques.

7. Agent de transfert selon la revendication 1 **caractérisé en ce qu'**il comporte un ou deux ponts disulfure.

8. Agent de transfert selon la revendication 1 **caractérisé en ce qu'**il comporte un pont disulfure positionné de sorte que sa réduction entraîne la perte d'une chaîne grasse aliphatique.

9. Agent de transfert selon la revendication 1 **caractérisé en ce qu'**il comporte un pont disulfure positionné de sorte que sa réduction entraîne la perte d'une chaîne dérivée d'un stéroïde présente dans la région lipophile.

10. Agent de transfert selon la revendication 1 **caractérisé en ce qu'**il est choisi parmi :

$$NH_2(CH_2)_3NH(CH_2)_4NH(CH_2)_3NHCH_2COCys[S\text{-}S\text{-}(CH_2)_4CH_3]\text{-}N[(CH_2)_{17}CH_3]_2 \quad (I);$$

$$NH_2(CH_2)_3NH(CH_2)_4NH(CH_2)_3NHCH_2COCys[S\text{-}S\text{-}(CH_2)_{17}CH_3]\text{-}NH(CH_2)_{17}CH_3 \quad (II);$$

$$NH_2(CH_2)_3NH(CH_2)_4NH(CH_2)_3NHCH_2COCys[S\text{-}S\text{-}(CH_2)_{11}CH_3]\text{-}NH(CH_2)_{17}CH_3 \quad (III);$$

$$NH_2(CH_2)_3NH(CH_2)_4NH(CH_2)_3NHCH_2COCys[S\text{-}S\text{-}(CH_2)_{11}CH_3]\text{-}N[(CH_2)_{17}CH_3]_2 \quad (IV);$$

$$NH_2(CH_2)_3NH(CH_2)_4NH(CH_2)_3NHCH_2COCys[S\text{-}S\text{-}Cholest\acute{e}rol]\text{-}NH(CH_2)_{17}CH_3 \quad (V);$$

$$[NH_2(CH_2)_3NH(CH_2)_4NH(CH_2)_3NHCH_2COCyNH(CH_2)_{17}CH_3]_2 \quad (VI);$$

11. Composition **caractérisée en ce qu'**elle comprend un agent de transfert tel que défini dans les revendications 1 à 10 et au moins un acide nucléique.

12. Composition selon la revendication 11 **caractérisée en ce que** l'acide nucléique est un acide désoxyribonucléique, ou bien un acide ribonucléique.

13. Composition selon la revendication 11 **caractérisée en ce que** l'acide nucléique est modifié chimiquement.

14. Composition selon la revendication 11 **caractérisée en ce que** l'acide nucléique est un antisens.

15. Composition selon la revendication 11 **caractérisée en ce que** l'acide nucléique comporte un gène d'intérêt thérapeutique.

16. Composition selon les revendications 11 à 15 **caractérisée en ce qu'**elle comprend en outre un adjuvant constitué par un ou plusieurs lipides neutres choisis parmi les lipides synthétiques ou naturels, zwitterioniques ou dépourvus de charge ionique dans les conditions physiologiques.

17. Composition selon la revendication 16 **caractérisée en ce que** le ou les lipides neutres sont le cholestérol ou bien des lipides à deux chaînes grasses de type dioleoylphosphatidyléthanolamine (DOPE), oléoylpalmitoylphosphatidyléthanolamine (POPE), les di-stéaroyl, -palmitoyl, -cholestéryl, -mirystoyl-phosphatidyléthanolamines ainsi que leurs dérivés N-méthylés 1 à 3 fois, les phosphatidylglycérols, les glycosyldiacylglycérols, les cérébrosides (tels que notamment les galactocérébrosides), les sphingolipides (tels que notamment les sphingomyélines), ou encore les asialogangliosides (tels que notamment les asialoGM1 et GM2).

18. Composition selon la revendication 11 **caractérisée en ce qu'**elle comporte en outre un adjuvant qui est ou qui comprend un composé dérivant d'une histone, d'une nucléoline, et/ou d'une protamine ou un composé constitué de motifs peptidiques (KTPKKAKKP) et/ou (ATPAKKAA) répétés de manière continue ou non, le nombre de motifs pouvant varier entre 2 et 10.

**19.** Composition selon la revendication 11 **caractérisée en ce qu'**elle associe en outre un élément de ciblage à l'agent de transfert.

**20.** Composition selon la revendication 19 **caractérisée en ce que** ledit élément de ciblage est choisi parmi les anticorps dirigés contre des molécules de la surface cellulaire, des ligands de recepteurs membranaires comme l'insuline, la transferrine, l'acide folique ou tout autre facteur de croissance, cytokines ou vitamines, des lectines, modifiées ou non, des protéines à motif RGD, des peptides contenant un tandem de motifs RGD, cyclique ou non, des peptides polylysine ainsi que des peptides ligands naturels ou synthétiques.

**21.** Composition selon la revendication 11 **caractérisée en ce qu'**elle incorpore en outre au moins un agent de surface non ionique de type poloxamères, les polyoxyéthylène alcools, le polyoxyéthylènenonylphényléther, ou bien les polyéthylèneglycol à tête polyéther benzylique dendritique.

**22.** Utilisation d'un agent de transfert tel que défini dans les revendications 1 à 10 pour le transfert *in vitro* d'acides nucléiques dans les cellules.

**23.** Méthode de transfert d'acides nucléiques dans les cellules **caractérisée en ce qu'**elle comprend les étapes suivantes :

(1) la mise en contact de l'acide nucléique avec un agent de transfert tel que défini dans les revendications 1 à 12 pour former un complexe acide nucléique / agent de transfert,
(2) la mise en contact *in vitro* des cellules avec le complexe formé en (1).

**24.** Méthode de transfert d'acides nucléiques dans les cellules selon la revendication 23 **caractérisée en ce que** dans le cas d'une composition comprenant un ou plusieurs autres agents de transfection et/ou un ou plusieurs adjuvants, l'étape (1) est précédée d'une étape de mise en contact des différents agents de transfection et/ou d'une étape de mise en contact de l'agent de transfection avec le ou les adjuvants.

**25.** Procédé de préparation d'une composition selon l'une des revendications 11 à 21 **caractérisé en ce que** l'on met en contact un acide nucléique avec un agent de transfert tel que défini dans les revendications 1 à 12 pour former un complexe acide nucléique/agent de transfert, précédé le cas échéant de la mise en contact préalable de l'agent de transfection avec un ou plusieurs adjuvants.

## Claims

**1.** Nucleic acid transfer agent comprising:

- at least one cationic hydrophilic region capable of combining in non-covalent manner with nucleic acids and,
- at least one lipophilic region constituted by at least one aliphatic fatty chain and one or more aliphatic chains, one or more steroid derivatives, a natural or synthetic lipid and/or optionally a combination of the latter,

these regions being linked to one another via an acid or amine group comprising hydolyzable functions, and moreover comprising at least one disulphide bridge **characterized in that** said cationic hydrophilic region is a polyamine or a polyaminoguanidine and that said disulphide bridge is positioned in the lipophilic region so as to generate a detergent amphiphilic molecule when it is reduced, or positioned such that its reduction leads to the resolution of said transfer agent when the latter is symmetrical.

**2.** Transfer agent according to claim 1 **characterized in that** the lipophilic region is constituted by at least two aliphatic fatty chains.

**3.** Transfer agent according to claim 1 **characterized in that** the lipophilic region is constituted by one aliphatic fatty chain and one steroid derivative.

**4.** Transfer agent according to claim 1 **characterized in that** the aliphatic fatty chain or chains are branched or linear alkyl chains comprising 10 to 22 carbon atoms, optionally saturated and/or fluorinated.

**5.** Transfer agent according to claim 1 **characterized in that** the steroid derivative is chosen from cholesterol, cholic

acid, and cholesterylamine.

6. Transfer agent according to claim 1 **characterized in that** the acid or amine group comprising hydrolyzable functions is chosen from the amide, carbamate, ester, ether groups and/or the aromatic rings.

7. Transfer agent according to claim 1 **characterized in that** it comprises one or two disulphide bridges.

8. Transfer agent according to claim 1 **characterized in that** it comprises one disulphide bridge positioned such that its reduction leads to the loss of an aliphatic fatty chain

9. Transfer agent according to claim 1 **characterized in that** it comprises one disulphide bridge positioned such that its reduction leads to the loss of a chain derived from a steroid present in the lipophilic region.

10. Transfer agent according to claim 1 **characterized in that** it is chosen from:

$$NH_2(CH_2)_3NH(CH_2)_4NH(CH_2)_3NHCH_2COCys[S\text{-}S\text{-}(CH_2)_4CH_3]\text{-}N[(CH_2)_{17}CH_3]_2 \quad (I);$$

$$NH_2(CH_2)_3NH(CH_2)_4NH(CH_2)_3NHCH_2COCys[S\text{-}S\text{-}(CH_2)_{17}CH_3]\text{-}NH(CH_2)_{17}CH_3 \quad (II);$$

$$NH_2(CH_2)_3NH(CH_2)_4NH(CH_2)_3NHCH_2COCys[S\text{-}S\text{-}(CH_2)_{11}CH_3]\text{-}NH[(CH_2)_{17}CH_3 \quad (III);$$

$$NH_2(CH_2)_3NH(CH_2)_4NH(CH_2)_3NHCH_2COCys[S\text{-}S\text{-}(CH_2)_{11}CH_3]\text{-}N[(CH_2)_{17}CH_3]_2 \quad (IV);$$

$$NH_2(CH_2)_3NH(CH_2)_4NH(CH_2)_3NHCH_2COCys[S\text{-}S\text{-}Cholesterol]\text{-}NH(CH_2)_{17}CH_3 \quad (V);$$

$$[NH_2(CH_2)_3NH(CH_2)_4NH(CH_2)_3NHCH_2COCysNH(CH_2)_{17}CH_3]_2 \quad (VI);$$

11. Composition **characterized in that** it comprises a transfer agent as defined in claims 1 to 10 and at least one nucleic acid.

12. Composition according to claim 11 **characterized in that** the nucleic acid is a deoxyribonucleic acid, or a ribonucleic acid.

13. Composition according to claim 11 **characterized in that** the nucleic acid is chemically modified.

14. Composition according to claim 11 **characterized in that** the nucleic acid is an anti-sense.

15. Composition according to claim 11 **characterized in that** the nucleic acid comprises a gene of therapeutic interest.

16. Composition according to claims 11 to 15 **characterized in that** it comprises moreover an adjuvant constituted by one or more neutral lipids chosen from the synthetic or natural lipids, zwitterionic or devoid of ionic charge under physiological conditions.

17. Composition according to claim 16 **characterized in that** the neutral lipid or lipids are cholesterol or lipids with two fatty chains of the dioleoylphosphatidylethanolamine (DOPE), oleoylpalmitoylphosphatidylethanolamine (POPE), di-stearoyl, -palmitoyl, -cholesteryl, -mirystoyl-phosphatidylethanolamine type, as well as their derivatives which are N-methylated 1 to 3 times, phosphatidylglycerols, glycosyldiacylglycerols, cerebrosides (such as in particular galactocerebrosides), sphingolipids (such as in particular sphingomyelins), or asialogangliosides (such as in particular asialo GM1 and GM2).

18. Composition according to claim 11 **characterized in that** it comprises moreover an adjuvant which is or which

comprises a compound deriving from a histone, a nucleolin, and/or a protamine or a compound constituted by peptide units (KTPKKAKKP) and/or (ATPAKKAA) repeated in a continuous manner or otherwise, the number of units being able to vary between 2 and 10.

**19.** Composition according to claim 11 **characterized in that** it moreover combines a targeting component with the transfer agent.

**20.** Composition according to claim 19 **characterized in that** said targeting component is chosen from the antibodies directed against cell-surface molecules, ligands of membrane receptors such as insulin, transferrin, folic acid or any other growth factor, cytokines or vitamins, lectins, modified or non-modified, proteins with the RGD unit, peptides containing a tandem of RGD units, cyclic or otherwise-cyclic, polylysine peptides as well as natural or synthetic ligand peptides.

**21.** Composition according to claim 11 **characterized in that** it moreover incorporates at least one non-ionic surface agent of the type comprising poloxamers, polyoxyethylene alcohols, polyoxyethylene nonyl phenyl ether, or polyethylene glycols with a dendritic benzyl polyether head.

**22.** Use of a transfer agent as defined in claims 1 to 10 for the *in vitro* transfer of nucleic acids into cells.

**23.** Method for transferring nucleic acids into cells **characterized in that** it comprises the following stages:

(1) bringing the nucleic acid into.contact with a transfer agent as defined in claims 1 to 10 in order to form a nucleic acid/transfer agent complex,
(2) bringing cells in contact *in vitro* with the complex formed in (1).

**24.** Method for transferring nucleic acids into cells according to claim 23 **characterized in that** in the case of a composition comprising one or more other transfection agents and/or one or more adjuvants, stage (1) is preceded by a stage of bringing the different transfection agents into contact, and/or a stage of bringing the transfection agent into contact with the adjuvant or adjuvants.

**25.** Method for preparing a composition according to one of claims 11 to 21 **characterized in that** a nucleic acid is brought into contact with a transfer agent as defined in claims 1 to 10 in order to form a nucleic acid/transfer agent complex, preceded, if appropriate, by the prior bringing of the transfection agent into contact with one or more adjuvants.

## Patentansprüche

**1.** Übertragungsmittel für Nucleinsäuren, umfassend:

- wenigstens einen kationischen hydrophilen Bereich, der sich nichtkovalent an Nucleinsäuren anlagern kann; und

- wenigstens einen lipophilen Bereich, der aus wenigstens einer aliphatischen Fettkette und einer oder mehreren aliphatischen Ketten, einem oder mehreren Steroidderivaten, einem natürlichen oder synthetischen Lipid und/oder gegebenenfalls einer Kombination derselben besteht;

wobei diese Bereiche über eine Säure- oder Amingruppe, die hydrolysierbare Funktionen trägt, miteinander verbunden sind und außerdem wenigstens eine Disulfidbrücke umfassen, **dadurch gekennzeichnet, dass** der kationische hydrophile Bereich ein Polyamin oder ein Polyaminoguanidin ist und dass sich die Disulfidbrücke im lipophilen Bereich befindet, so dass bei ihrer Reduktion ein amphiphiles Tensidmolekül entsteht, oder aber so positioniert ist, dass ihre Reduktion zur Halbierung des Übertragungsmittels führt, wenn dieses symmetrisch ist.

**2.** Übertragungsmittel gemäß Anspruch 1, **dadurch gekennzeichnet, dass** der lipophile Bereich aus wenigstens zwei aliphatischen Fettketten besteht.

**3.** Übertragungsmittel gemäß Anspruch 1, **dadurch gekennzeichnet, dass** der lipophile Bereich aus einer aliphatischen Fettkette und einem Steroidderivat besteht.

4. Übertragungsmittel gemäß Anspruch 1, **dadurch gekennzeichnet, dass** die aliphatische(n) Fettkette(n) verzweigte oder lineare Alkylketten sind, die 10 bis 22 Kohlenstoffatome umfassen und gegebenenfalls gesättigt und/oder fluoriert sind.

5. Übertragungsmittel gemäß Anspruch 1, **dadurch gekennzeichnet, dass** das Steroidderivat aus Cholesterin, Cholsäure und Cholesterylamin ausgewählt ist.

6. Übertragungsmittel gemäß Anspruch 1, **dadurch gekennzeichnet, dass** die Säure- oder Amingruppe, die hydrolysierbare Funktionen trägt, aus Amid-, Carbamat-, Ester-, Ethergruppen und/oder aromatischen Cyclen ausgewählt ist.

7. Übertragungsmittel gemäß Anspruch 1, **dadurch gekennzeichnet, dass** es eine oder zwei Disulfidbrücken trägt.

8. Übertragungsmittel gemäß Anspruch 1, **dadurch gekennzeichnet, dass** es eine Disulfidbrücke trägt, die so positioniert ist, dass ihre Reduktion zum Verlust einer aliphatischen Fettkette führt.

9. Übertragungsmittel gemäß Anspruch 1, **dadurch gekennzeichnet, dass** es eine Disulfidbrücke trägt, die so positioniert ist, dass ihre Reduktion zum Verlust einer Kette führt, die von einem Steroid abgeleitet ist und sich im lipophilen Bereich befindet.

10. Übertragungsmittel gemäß Anspruch 1, **dadurch gekennzeichnet, dass** es ausgewählt ist aus:

$$NH_2(CH_2)_3NH(CH_2)_4NH(CH_2)_3NHCH_2COCys[S\text{-}S\text{-}(CH_2)_4CH_3]\text{-}N[(CH_2)_{17}CH_3]_2 \quad (I);$$

$$NH_2(CH_2)_3NH(CH_2)_4NH(CH_2)_3NHCH_2COCys[S\text{-}S\text{-}(CH_2)_{17}CH_3]\text{-}NH(CH_2)_{17}CH_3 \quad (II);$$

$$NH_2(CH_2)_3NH(CH_2)_4NH(CH_2)_3NHCH_2COCys[S\text{-}S\text{-}(CH_2)_{11}CH_3]\text{-}NH(CH_2)_{17}CH_3 \quad (III);$$

$$NH_2(CH_2)_3NH(CH_2)_4NH(CH_2)_3NHCH_2COCys[S\text{-}S\text{-}(CH_2)_{11}CH_3]\text{-}N[(CH_2)_{17}CH_3]_2 \quad (IV);$$

$$NH_2(CH_2)_3NH(CH_2)_4NH(CH_2)_3NHCH_2COCys[S\text{-}S\text{-}Cholesterin]\text{-}NH(CH_2)_{17}CH_3 \quad (V);$$

$$[NH_2(CH_2)_3NH(CH_2)_4NH(CH_2)_3NHCH_2COCysNH(CH_2)_{17}CH_3]_2 \quad (VI).$$

11. Zusammensetzung, **dadurch gekennzeichnet, dass** sie ein Übertragungsmittel, wie es in den Ansprüchen 1 bis 10 definiert ist, und wenigstens eine Nucleinsäure umfasst.

12. Zusammensetzung gemäß Anspruch 11, **dadurch gekennzeichnet, dass** die Nucleinsäure eine Desoxyribonucleinsäure oder eine Ribonucleinsäure ist.

13. Zusammensetzung gemäß Anspruch 11, **dadurch gekennzeichnet, dass** die Nucleinsäure chemisch modifiziert ist.

14. Zusammensetzung gemäß Anspruch 11, **dadurch gekennzeichnet, dass** die Nucleinsäure eine Antisense-Nucleinsäure ist.

15. Zusammensetzung gemäß Anspruch 11, **dadurch gekennzeichnet, dass** die Nucleinsäure ein Gen von therapeutischem Interesse trägt.

16. Zusammensetzung gemäß den Ansprüchen 11 bis 15, **dadurch gekennzeichnet, dass** sie außerdem ein Adjuvans umfasst, das aus einem oder mehreren neutralen Lipiden besteht, die aus synthetischen oder natürlichen

Lipiden ausgewählt sind, welche unter physiologischen Bedingungen zwitterionisch sind oder keine Ionenladung tragen.

**17.** Zusammensetzung gemäß Anspruch 16, **dadurch gekennzeichnet, dass** es sich bei dem bzw. den neutralen Lipiden um Cholesterin oder Lipide mit zwei Fettketten des Typs Dioleylphosphatidylethanolamin (DOPE), Oleoylpalmitoylphosphatidylethanolamin (POPE), Distearoyl-, -palmitoyl-, -cholesteryl-, -myristoylphosphatidylethanolamine sowie ihre ein- bis dreifach N-methylierten Derivate, Phosphatidylglycerine, Glycosyldiacylglycerine, Cerebroside (wie insbesondere Galactocerebroside), Sphingolipide (wie insbesondere Sphingomyeline) oder aber Asialoganglioside (wie insbesondere Asialo-GM1 und -GM2) handelt.

**18.** Zusammensetzung gemäß Anspruch 11, **dadurch gekennzeichnet, dass** sie außerdem ein Adjuvans umfasst, bei dem es sich um Folgendes handelt bzw. das Folgendes umfasst: eine Verbindung, die von einem Histon, einem Nucleolin und/oder einem Protamin abgeleitet ist, oder eine Verbindung, die aus Peptidmotiven (KTPKKAKKP) und/oder (ATPAKKAA) besteht, die sich kontinuierlich wiederholen oder auch nicht, wobei die Zahl der Motive zwischen 2 und 10 variieren kann.

**19.** Zusammensetzung gemäß Anspruch 11, **dadurch gekennzeichnet, dass** sie außerdem ein Zielsteuerungselement für das Übertragungsmittel umfasst.

**20.** Zusammensetzung gemäß Anspruch 19, **dadurch gekennzeichnet, dass** das Zielsteuerungselement ausgewählt ist aus Antikörpern gegen Moleküle der Zelloberfläche, Liganden von Membranrezeptoren, wie Insulin, Transferrin, Folsäure oder jeder andere Wachstumsfaktor, Cytokinen oder Vitaminen, modifizierten oder unmodifizierten Lectinen, Proteinen mit RGD-Motiv, Peptiden, die ein cyclisches oder nichtcyclisches Tandem von RGD-Motiven enthalten, Polylysin-Peptiden sowie natürlichen oder synthetischen Ligandenpeptiden.

**21.** Zusammensetzung gemäß Anspruch 11, **dadurch gekennzeichnet, dass** sie außerdem wenigstens ein nichtionisches Tensid des Typs Poloxamere, Polyoxyethylenalkohole, Polyoxyethylennonylphenylether oder auch Polyethylenglycol mit einem dendritischen Kopf aus benzylischem Polyether umfassen.

**22.** Verwendung eines Übertragungsmittels, wie es in den Ansprüchen 1 bis 10 definiert ist, für die in-vitro-Übertragung von Nucleinsäuren in Zellen.

**23.** Verfahren zur Übertragung von Nucleinsäuren in Zellen, **dadurch gekennzeichnet, dass** es die folgenden Schritte umfasst:

(1) In-Kontakt-Bringen der Nucleinsäure mit einem Übertragungsmittel, wie es in den Ansprüchen 1 bis 10 definiert ist, unter Bildung eines Nucleinsäure/Übertragungsmittel-Komplexes;

(2) In-Kontakt-Bringen der Zellen mit dem unter (1) gebildeten Komplex in vitro.

**24.** Verfahren zur Übertragung von Nucleinsäuren in Zellen gemäß Anspruch 23, **dadurch gekennzeichnet, dass** im Falle einer Zusammensetzung, die ein oder mehrere andere Transfektionsmittel und/oder ein oder mehrere Adjuvantien umfasst, dem Schritt (1) ein Schritt, bei dem die anderen Transfektionsmittel in Kontakt gebracht werden, und/oder ein Schritt, bei dem das Transfektionsmittel mit dem oder den Adjuvantien in Kontakt gebracht wird, vorausgeht.

**25.** Verfahren zur Herstellung einer Zusammensetzung gemäß einem der Ansprüche 11 bis 21, **dadurch gekennzeichnet, dass** man eine Nucleinsäure mit einem Übertragungsmittel, wie es in den Ansprüchen 1 bis 10 definiert ist, in Kontakt bringt, so dass sich ein Nucleinsäure/Übertragungsmittel-Komplex bildet, wobei diesem Schritt gegebenenfalls das vorherige In-Kontakt-Bringen des Transfektionsmittels mit einem oder mehreren Adjuvantien vorausgeht.

# FIGURE 1/9

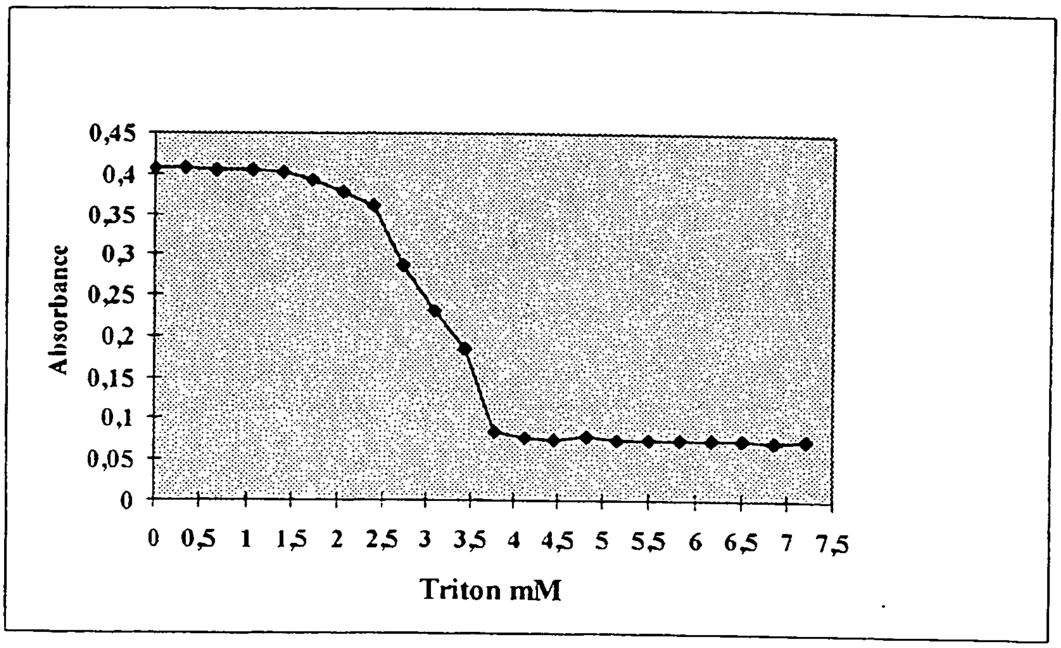
0,45
0,4
0,35
0,3
0,25
0,2
0,15
0,1
0,05
0
Absorbance
0 0,5 1 1,5 2 2,5 3 3,5 4 4,5 5 5,5 6 6,5 7 7,5
Triton mM

# FIGURE 2/9

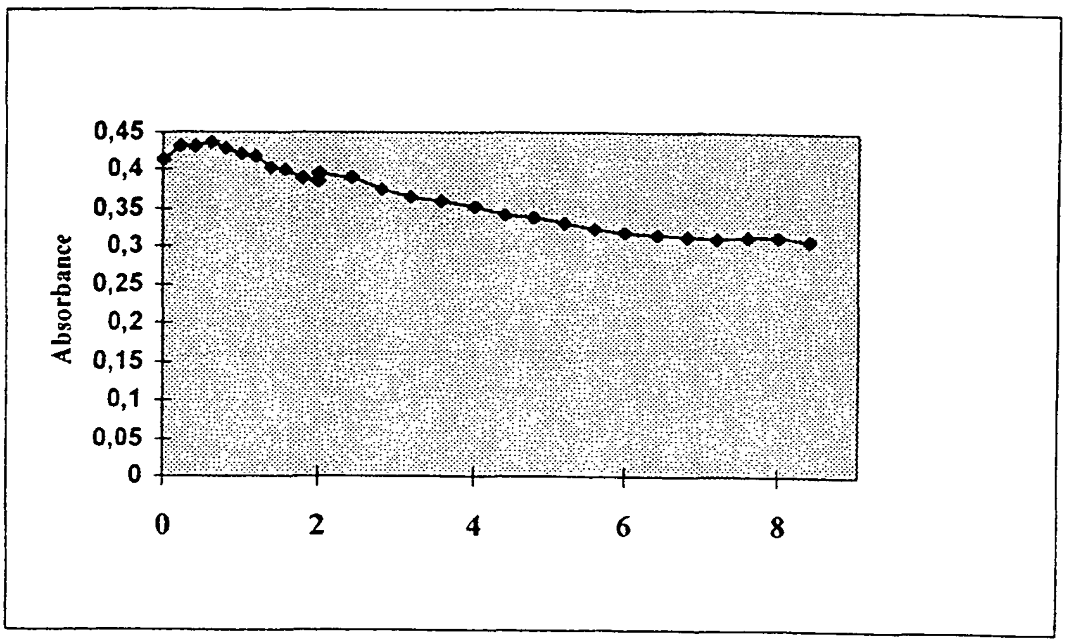

## FIGURE 3/9

| | nmol/µg d'ADN | RLU/5µl extrait cellulaire/10s | RLU/µg protéine/10s |
|---|---|---|---|
| lipide cationique de référence REF | 2 | 2,5 E+03 | 1,7 E+03 |
| | 4 | 3,7 E+03 | 2,9 E+03 |
| | 6 | 2,6 E+04 | 1,8 E+04 |
| | 8 | 3,1 E+04 | 2,2 E+04 |
| Agent (VI) | 2 | 1,1 E+03 | 7,7 E+02 |
| | 4 | 7,4 E+04 | 5,9 E+04 |
| | 6 | 1,1 E+05 | 9,6 E+04 |
| | 8 | 5,9 E+04 | 5,3 E+04 |

Tests sur les cellules HepG2

# FIGURE 4/9

| | nmol/µg d'ADN | SANS SERUM RLU/5µl extrait cellulaire/10s | SANS SERUM RLU/µg protéine/10s | AVEC SERUM RLU/5µl extrait cellulaire/10s | AVEC SERUM RLU/µg protéine/10s |
|---|---|---|---|---|---|
| lipide cationique de référence REF | 2 | 2,5 E+03 | 1,7 E+03 | 3,1 E+03 | 2,6 E+03 |
| | 4 | 3,7 E+03 | 2,9 E+03 | 5,3 E+01 | 4,1 E+01 |
| | 6 | 2,6 E+04 | 1,8 E+04 | 5,2 E+02 | 4,7 E+02 |
| | 8 | 3,1 E+04 | 2,2 E+04 | 5,3 E+02 | 4,0 E+02 |
| Agent (I) | 2 | 3,4 E+03 | 2,4 E+03 | 1,4 E+03 | 1,2 E+03 |
| | 4 | 4,7 E+03 | 3,7 E+03 | 2,6 E+03 | 2,3 E+03 |
| | 6 | 1,1 E+04 | 8,7 E+03 | 8,3 E+03 | 7,2 E+03 |
| | 8 | 4,4 E+04 | 3,3 E+04 | 1,5 E+04 | 1,2 E+04 |
| Agent (IV) | 2 | 5,9 E+03 | 4,4 E+03 | 2,5 E+03 | 2,2 E+03 |
| | 4 | 1,3 E+05 | 1,0 E+05 | 3,0 E+04 | 2,6 E+04 |
| | 6 | 2,2 E+05 | 1,7 E+05 | 4,4 E+04 | 4,1 E+04 |
| | 8 | 2,2 E+05 | 1,8 E+05 | 6,7 E+04 | 5,7 E+04 |

Tests sur les cellules HepG2

| | nmol/µg d'ADN | SANS SERUM RLU/5µl extrait cellulaire/10s | SANS SERUM RLU/µg protéine/10s | AVEC SERUM RLU/5µl extrait cellulaire/10s | AVEC SERUM RLU/µg protéine/10s |
|---|---|---|---|---|---|
| lipide cationique de référence REF | 2 | 2,9 E+05 | 1,6 E+05 | 1,5 E+05 | 7,8 E+04 |
| | 4 | 2,2 E+06 | 1,7 E+06 | 6,8 E+03 | 3,5 E+03 |
| | 6 | 3,4 E+06 | 2,8 E+06 | 1,7 E+04 | 9,2 E+03 |
| | 8 | 4,3 E+06 | 3,5 E+06 | 2,8 E+04 | 1,5 E+04 |
| Agent (I) | 2 | 7,0 E+05 | 4,2 E+05 | 1,7 E+06 | 9,8 E+05 |
| | 4 | 5,2 E+06 | 3,3 E+06 | 7,9 E+06 | 4,7 E+06 |
| | 6 | 3,7 E+06 | 2,6 E+06 | 5,2 E+06 | 3,2 E+06 |
| | 8 | 3,3 E+06 | 2,3 E+06 | 4,5 E+06 | 2,7 E+06 |
| Agent (IV) | 2 | 5,2 E+05 | 3,4 E+05 | 4,3 E+05 | 2,8 E+05 |
| | 4 | 3,7 E+06 | 2,2 E+06 | 3,7 E+06 | 2,4 E+06 |
| | 6 | 7,1 E+06 | 4,7 E+06 | 5,1 E+06 | 4,1 E+06 |
| | 8 | 8,2 E+06 | 5,6 E+06 | 6,8 E+06 | 5,7 E+06 |

Tests sur les cellules HeLa

**FIGURE 5/9**

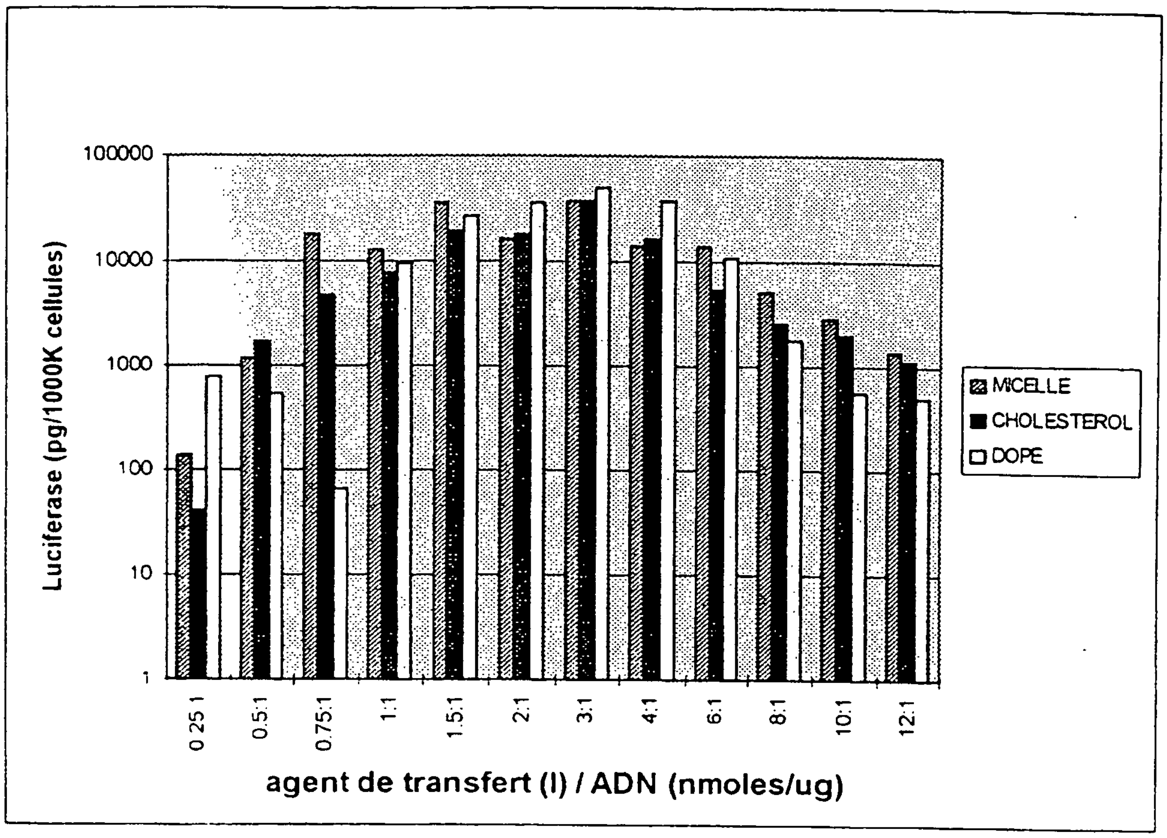
100000
10000
1000
100
10
1
Luciferase (pg/1000K cellules)
0.25:1
0.5:1
0.75:1
1:1
1.5:1
2:1
3:1
4:1
6:1
8:1
10:1
12:1
agent de transfert (I) / ADN (nmoles/ug)
MICELLE
CHOLESTEROL
DOPE

FIGURE 6/9

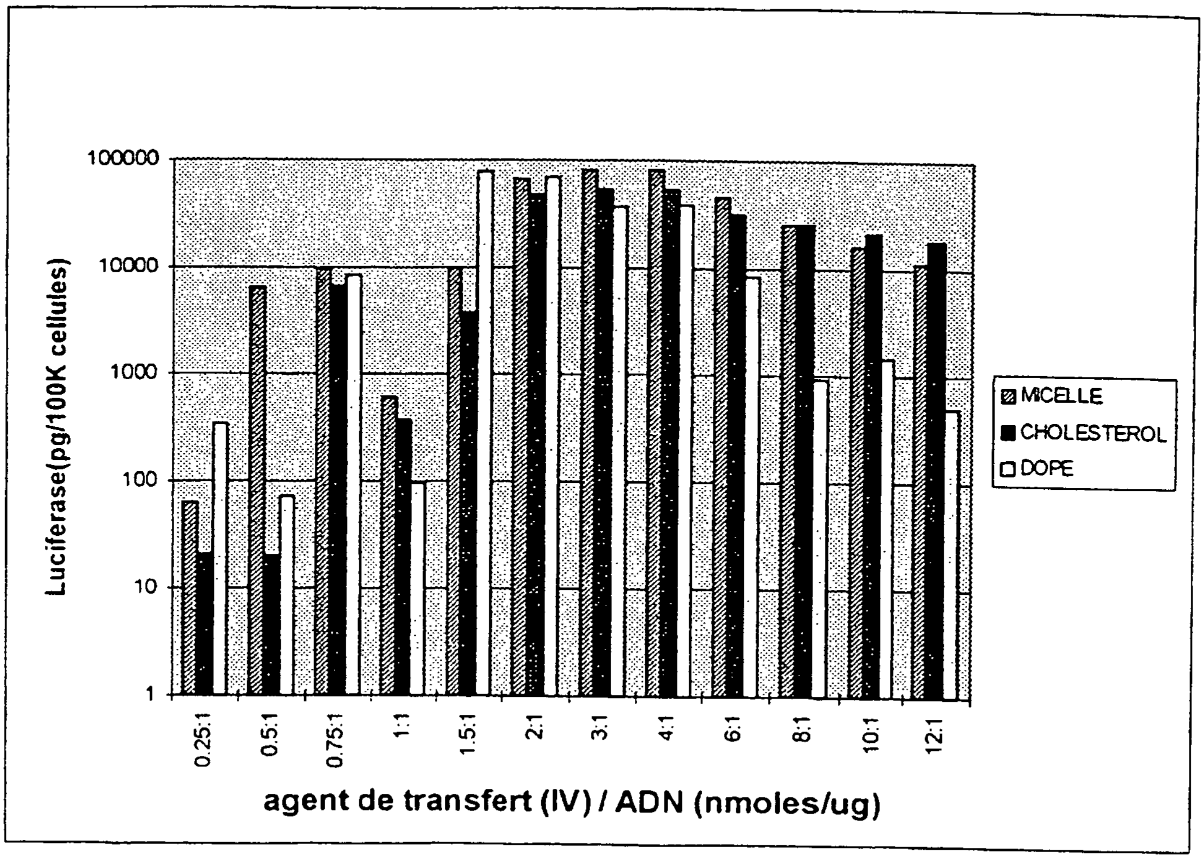
Luciferase(pg/100K cellules)
100000
10000
1000
100
10
1
0.25:1
0.5:1
0.75:1
1:1
1.5:1
2:1
3:1
4:1
6:1
8:1
10:1
12:1
agent de transfert (IV) / ADN (nmoles/ug)
MICELLE
CHOLESTEROL
DOPE

## FIGURE 7/9

| | nmol/µg d'ADN | SANS SERUM RLU/5µl extrait cellulaire/10s | SANS SERUM RLU/µg protéine/10s | AVEC SERUM RLU/5µl extrait cellulaire/10s | AVEC SERUM RLU/µg protéine/10s |
|---|---|---|---|---|---|
| lipide cationique de référence REF | 2 | 2,5 E+03 | 1,7 E+03 | 3,1 E+03 | 2,6 E+03 |
| | 4 | 3,7 E+03 | 2,9 E+03 | 5,3 E+01 | 4,1 E+01 |
| | 6 | 2,6 E+04 | 1,8 E+04 | 5,2 E+02 | 4,7 E+02 |
| | 8 | 3,1 E+04 | 2,2 E+04 | 5,3 E+02 | 4,0 E+02 |
| Agent (II) | 2 | 4,3 E+01 | 3,4 E+01 | 1,5 E+01 | 1,8 E+01 |
| | 4 | 1,7 E+03 | 1,3 E+03 | 1,1 E+02 | 1,3 E+02 |
| | 6 | 1,7 E+04 | 1,6 E+04 | 2,2 E+03 | 3,0 E+03 |
| | 8 | 9,1 E+04 | 7,6 E+04 | 1,6 E+04 | 1,6 E+04 |

Tests sur les cellules HepG2

## FIGURE 8/9

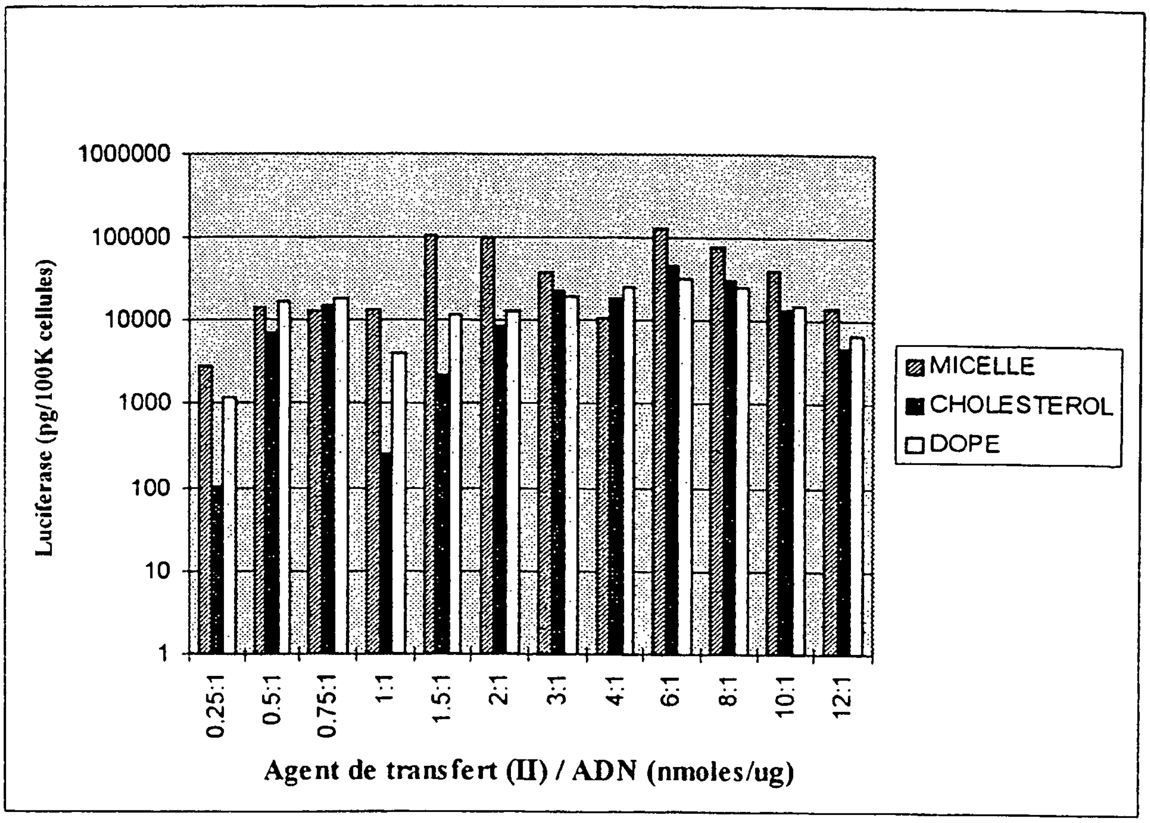
1000000
100000
10000
1000
100
10
1
Luciferase (pg/100K cellules)
0.25:1
0.5:1
0.75:1
1:1
1.5:1
2:1
3:1
4:1
6:1
8:1
10:1
12:1
Agent de transfert (II) / ADN (nmoles/ug)
MICELLE
CHOLESTEROL
DOPE

# FIGURE 9/9

| | nmol/µg d'ADN | SANS SERUM RLU5µl extrait cellulaire/10s | SANS SERUM RLU/µg protéine/10s | AVEC SERUM RLU/5µl extrait cellulaire/10s | AVEC SERUM RLU/µg protéine/10s |
|---|---|---|---|---|---|
| lipide cationique de référence REF | 2 | 5,1 E+05 | 1,0 E+06 | 1,2 E+05 | 2,2 E+05 |
| | 4 | 1,2 E+05 | 2,3 E+05 | 1,0 E+04 | 1,9 E+04 |
| | 6 | 5,8 E+04 | 1,3 E+05 | 5,8 E+03 | 1,4 E+04 |
| | 8 | 2,1 E+04 | 3,6 E+04 | 3,3 E+03 | 7,3 E+03 |
| Agent (V) | 2 | 2,0 E+04 | 3,9 E+04 | 2,6 E+04 | 4,6 E+04 |
| | 4 | 1,3 E+05 | 2,3 E+05 | 5,4 E+04 | 9,0 E+04 |
| | 6 | 1,8 E+05 | 3,3 E+05 | 6,1 E+04 | 1,1 E+05 |
| | 8 | 3,2 E+05 | 6,0 E+05 | 7,1 E+04 | 1,3 E+05 |

Tests sur les cellules HepG2

| | nmol/µg d'ADN | SANS SERUM RLU/5µl extrait cellulaire/10s | SANS SERUM RLU/µg protéine/10s | AVEC SERUM RLU/5µl extrait cellulaire/10s | AVEC SERUM RLU/µg protéine/10s |
|---|---|---|---|---|---|
| lipide cationique de référence REF | 2 | 2,2 E+06 | 1,2 E+06 | 3,0 E+06 | 1,5 E+06 |
| | 4 | 5,9 E+06 | 3,4 E+06 | 5,1 E+03 | 2,0 E+03 |
| | 6 | 4,0 E+06 | 2,6 E+06 | 9,5 E+03 | 3,8 E+03 |
| | 8 | 1,2 E+06 | 6,8 E+05 | 4,2 E+03 | 1,6 E+03 |
| Agent (V) | 2 | 1,9 E+05 | 8,2 E+04 | 2,9 E+05 | 1,2 E+05 |
| | 4 | 7,9 E+05 | 3,3 E+05 | 1,0 E+06 | 4,1 E+05 |
| | 6 | 1,4 E+06 | 6,4 E+05 | 2,0 E+06 | 8,4 E+05 |
| | 8 | 3,0 E+06 | 1,5 E+06 | 5,0 E+06 | 2,1 E+06 |

Tests sur les cellules HeLa